# EUROPEAN PATENT APPLICATION

(11) **EP 3 085 793 A1**
(43) Date of publication of application: **26.10.2016**
(21) Application number: 16167951.9
(22) Date of filing: 28.08.2012
(51) Int. Cl.: C12Q 1/68

(54) **METHOD FOR PREDICTING RISK OF HYPERTENSION ASSOCIATED WITH ANTI-ANGIOGENESIS THERAPY**

(30) Priority: 31.08.2011 EP 11179500
(62) Divisional of application: 12750601.2
(71) Applicant: F. Hoffmann-La Roche AG, 4070 Basel (CH); VIB vzw, 9052 Gent (BE); Life Sciences Research Partners VZW, 3000 Leuven (BE)
(72) Inventor: DE HAAS, Sanne Lysbet, 4056 Basel (CH); DELMAR, Paul, 4057 Basel (CH); LAMBRECHTS, Diether, 3010 Kessel-lo (Leuven) (BE); SCHERER, Stefan, Jersey City, NJ 07302 (US)
(74) Representative: Brodbeck, Michel

(57) **Abstract**

The invention is concerned with a method of predicting a patient's susceptibility to developing hypertension associated with an anti-angiogenesis treatment, such as bevacizumab, by determing the genotype of KDR gene. The invention further relates to a pharmaceutical composition comprising an angiogenesis inhibitor, such as bevacizumab, for the treatment of a patient suffering from cancer based on the genotype of KDR gene. The invention further relates to a method for reducing the risk of hypertension associated with an anti-angiogenesis therapy, such as bevacizumab, in a patient suffering from cancer by determining the genotype of KDR gene.

## Description

### Field of the Invention

The present invention is directed to methods of predicting the risk of hypertension in patients undergoing anti-angiogenesis therapy, including a therapy with bevacizumab.

### Background of the Invention

Angiogenesis contributes to benign and malignant diseases such as cancer development and, especially in cancer, is necessary for primary tumor growth, invasiveness and metastasis. In order to grow, a tumor must undergo an angiogenic switch. Vascular endothelial growth factor (VEGF) is required to induce this angiogenic switch. VEGF and the genes in the VEGF pathway are considered important mediators of cancer progression. The VEGF gene family includes the VEGF gene, also referred to as VEGFA, homologues to VEGF including, placenta growth factor (PlGF), VEGFB, VEGFC, VEGFD, the VEGF receptors, including VEGFR-1 and VEGFR-2 (also referred to as FLT1 and FLK1/KDR, respectively), the VEGF inducers, including hypoxia-inducible factors HIF1α, HIF2 α, and the oxygen sensors PHD1, PHD2 and PHD3.

The importance of this pathway in cancer cell growth and metastasis has led to the development of anti-angiogenesis agents for use in cancer therapy. These therapies include, among others, bevacizumab, pegaptanib, sunitinib, sorafenib and vatalanib. Despite significantly prolonged survival obtained with angiogenesis inhibitors, such as bevacizumab, patients still succumb to cancer. Further, not all patients respond to angiogenesis inhibitor therapy. The mechanism underlying the non-responsiveness remains unknown. Moreover, angiogenesis inhibitor therapy is associated with side effects, such as gastrointestinal perforation, thrombosis, bleeding, hypertension and proteinuria.

Accordingly, there is a need for methods of determining which patients respond particular well to angiogenesis inhibitor therapy and/or which patients are susceptible to side effects associated with anti-angiogenesis treatments.

### Summary of the Invention

The present invention relates to a method of determining susceptibility of a patient to developing hypertension associated with a therapy by an angiogenesis inhibitor, such as bevacizumab, by determining the genotype at polymorphism rs2305949 (SEQ ID NO. 3) in KDR and/or rs4444903 (SEQ ID NO. 4) in EGF, which is associated with a reduced risk of hypertension. The present invention further relates to a pharmaceutical composition comprising an angiogenesis inhibitor, such as bevacizumab, for the treatment of a patient suffering from cancer and having the genotype at polymorphism rs2305949 (SEQ ID NO. 3) and/or rs4444903 (SEQ ID NO. 4) associated with a reduced risk of hypertension. The present invention further relates to a method for reducing the risk of hypertension associated with an anti-angiogenesis therapy, such as bevacizumab, in a patient suffering from cancer by detecting the genotype at rs2305949 (SEQ ID NO. 3) and/or rs4444903 (SEQ ID NO. 4) which is linked with a reduced risk of hypertension.

One embodiment of the invention provides methods of determining susceptibility of a patient to developing hypertension associated with a therapy comprising an angiogenesis inhibitor comprising bevacizumab or an antibody that binds essentially the same epitope on VEGF as bevacizumab. The methods comprise (a) determining in a sample derived from a patient suffering from cancer the genotype at polymorphism rs2305949 (SEQ ID NO. 3), and (b) identifying a patient as more or less susceptible to developing hypertension associated with a therapy by an angiogenesis inhibitor comprising bevacizumab or an antibody that binds essentially the same epitope on VEGF as bevacizumab based on said genotype, wherein the presence of CC genotype at polymorphism rs2305949 (SEQ ID NO. 3) indicates that said patient is more susceptible to developing hypertension than a patient having a genotype of CT or TT at polymorphism rs2305949 (SEQ ID NO. 3), or the presence of CT or TT genotype at polymorphism rs2305949 (SEQ ID NO. 3) indicates that said patient is less susceptible to developing hypertension than a patient having a genotype of CC at polymorphism rs2305949 (SEQ ID NO. 3). In some embodiments, the methods *are in vitro Methods.* In some embodiments, the therapy further comprises a chemotherapeutic agent or chemotherapy regimen.

In some embodiments, the angiogenesis inhibitor is administered with one or more agents selected from the group consisting of taxanes, interferon alpha, 5-fluorouracil, leucovorin, irinotecan, gemcitabine-erlotinib and platinum-based chemotherapeutic agents. In some embodiments, the cancer is pancreatic cancer, renal cell cancer, colorectal cancer, breast cancer or lung cancer. In some embodiments, the sample is a blood sample. In some embodiments, the genotype is determined by means of MALDI-TOF mass spectrometry. In some embodiments, the methods further comprise administering the therapy to the patient.

Another embodiment of the invention provides pharmaceutical compositions comprising an angiogenesis inhibitor as defined herein for the treatment of a patient in need thereof, wherein said patient has been determined to be less susceptible to developing hypertension associated with a therapy comprising by the angiogenesis inhibitor in accordance with the method described herein.

A further embodiment of the invention provides kits for carrying out the methods described herein. The kits comprise oligonucleotides capable of determining the genotype at polymorphism rs2305949 (SEQ ID NO. 3).

Even another embodiment of the invention provides methods of reducing the risk of developing hypertension associated with a therapy comprising an angiogenesis inhibitor comprising bevacizumab or an antibody that binds essentially the same epitope on VEGF as bevacizumab. The methods comprise (a) determining in a sample derived from a patient suffering from cancer the genotype at polymorphism rs2305949 (SEQ ID NO. 3); (b) identifying a patient as less susceptible to developing hypertensin associated with a therapy by an angiogenesis inhibitor comprising bevacizumab or an antibody that binds essentially the same epitope on VEGF as bevacizumab, wherein the presence of CT or TT genotype at polymorphism rs2305949 (SEQ ID NO. 3) indicates that said patient is less susceptible to developing hypertension than a patient having a genotype of CC at polymorphism rs2305949 (SEQ ID NO. 3); and (c) administering said angiogenesis inhibitor to a patient with the CT or TT genotype at polymorphism rs2305949 (SEQ ID NO. 3) identified as less susceptible to developing hypertension in accordance with (b). In some embodiments, the therapy further comprises a chemotherapeutic agent or chemotherapy regimen. In some embodiments, the angiogenesis inhibitor is administered with one or more agents selected from the group consisting of taxanes, interferon alpha, 5-fluorouracil, leucovorin, irinotecan, gemcitabine-erlotinib and platinum-based chemotherapeutic agents. In some embodiments, the cancer is pancreatic cancer, renal cell cancer, colorectal cancer, breast cancer or lung cancer. In some embodiments, the sample is a blood sample. In some embodiments, the genotype is determined by means of MALDI-TOF mass spectrometry.

Yet another embodiment of the invention provies methods of treating a patient. The methods comprise administering to the patient therapy comprising an angiogenensis inhibitor comprising bevacizumab or an antibody that binds essentially the same epitope on VEGF as bevacizumab wherein the patient genotype at polymorphism rs2305949 (SEQ ID NO. 3) has been determined to be CT or TT.

Even a further embodiment of the invention provides methods of determining susceptibility of a patient to developing hypertension associated with a therapy comprising an angiogenesis inhibitor comprising bevacizumab or an antibody that binds essentially the same epitope on VEGF as bevacizumab The methods comprise (a) determining in a sample derived from a patient suffering from cancer the genotype at polymorphism rs4444903 (SEQ ID NO. 4), and (b) identifying a patient as more or less susceptible to developing hypertension associated with a therapy by an angiogenesis inhibitor comprising bevacizumab or an antibody that binds essentially the same epitope on VEGF as bevacizumab based on said genotype, wherein the presence of GA genotype at polymorphism rs rs4444903 (SEQ ID NO. 4) indicates that said patient is more susceptible to developing hypertension than a patient having a genotype of GG or AA at polymorphism rs4444903 (SEQ ID NO. 4), or the presence of GG or AA genotype at polymorphism rs4444903 (SEQ ID NO. 4) indicates that said patient is less susceptible to developing hypertension than a patient having a genotype of GA at polymorphism rs4444903 (SEQ ID NO. 4). In some embodiments, the methods are *in vitro* methods. In some embodiments, the therapy further comprises a chemotherapeutic agent or chemotherapy regimen. In some embodiments, the angiogenesis inhibitor is administered with one or more agents selected from the group consisting of taxanes, interferon alpha, 5-fluorouracil, leucovorin, irinotecan, gemcitabine-erlotinib and platinum-based chemotherapeutic agents. In some embodiments, the cancer is pancreatic cancer, renal cell cancer, colorectal cancer, breast cancer or lung cancer. In some embodiments, the sample is a blood sample. In some embodiments, the genotype is determined by means of MALDI-TOF mass spectrometry. In some embodiments the methods further comprise administering the therapy to the patient.

A further embodiment of the invention provides pharmaceutical compositions comprising an angiogenesis inhibitor as defined herein for the treatment of a patient in need thereof, wherein said patient has been determined to be less susceptible to developing hypertension associated with a therapy by the angiogenesis inhibitor in accordance with the methods described herein.

Yet another embodiment of the invention provides kits for carrying out the methods described herein. The kits comprise oligonucleotides capable of determining the genotype at polymorphism rs4444903 (SEQ ID NO. 4).

A further embodiment of the invention provides methods of reducing the risk of developing hypertension associated with a therapy comprising an angiogenesis inhibitor comprising bevacizumab or an antibody that binds essentially the same epitope on VEGF as bevacizumab. The methods comprise (a) determining in a sample derived from a patient suffering from cancer the genotype at polymorphism rs4444903 (SEQ ID NO. 4); (b) identifying a patient as less susceptible to developing hypertensin associated with a therapy by an angiogenesis inhibitor comprising bevacizumab or an antibody that binds essentially the same epitope on VEGF as bevacizumab, wherein the presence of GG or AA genotype at polymorphism rs4444903 (SEQ ID NO. 4) indicates that said patient is less susceptible to developing hypertension than a patient having a genotype of GA at polymorphism rs4444903 (SEQ ID NO. 4); and (c) administering said angiogenesis inhibitor to a patient with the GG or AA genotype at polymorphism rs4444903 (SEQ ID NO. 4) identified as less susceptible to developing hypertension in accordance with (b). In some embodiments, the therapy further comprises a chemotherapeutic agent or chemotherapy regimen. In some embodiments, the angiogenesis inhibitor is administered with one or more agents selected from the group consisting of taxanes, interferon alpha, 5-fluorouracil, leucovorin, irinotecan, gemcitabine-erlotinib and platinum-based chemotherapeutic agents. In some embodiments, the cancer is pancreatic cancer, renal cell cancer, colorectal cancer, breast cancer or lung cancer. In some embodiments, the sample is a blood sample. In some embodiments, the genotype is determined by means of MALDI-TOF mass spectrometry.

Another embodiment of the invention provides methods of treating a patient. The methods comprising administering to the patient therapy comprising an angiogenensis inhibitor comprising bevacizumab or an antibody that binds essentially the same epitope on VEGF as bevacizumab wherein the patient genotype at polymorphism rs4444903 (SEQ ID NO. 4) has been determined to be GG or AA.

### Brief Description of the Drawings

Figure 1: Endpoint distributions of clinical data. Kaplan-Meier plot of Progression-free Survival (PFS).
Figure 2: Endpoint distributions of clinical data. Kaplan-Meier plot of Overall Survival (OS).
Figure 3: Endpoint distributions of clinical data. Bar chart of Best Overall Response (BOR). Rates of BOR were 49% in BEV-treated subjects and 46% in PBO-treated subjects.
Figure 4: Endpoint distributions of clinical data. Bar chart of Hypertension not unrelated to study drug. Rates of hypertension were 18% in BEV-treated subjects and 7% in PBO-treated subjects.
Figure 5: Association analysis results for VEGFA and PFS in the Leuven efficacy panel analysis.
Figure 6: Forest plot for rs699946 (SEQ ID NO. 1) in VEGFA when tested for association against PFS.
Figure 7: Forest plot for the association of rs12505758 (SEQ ID NO. 2) with OS in white BEV-treated subjects.
Figure 8: Kaplan Meier plots for association between rs12505758 (SEQ ID NO. 2) and OS.
Figure 9: Hypertension frequencies in correlation to rs2305949 (SEQ ID NO. 3) (KDR).
Figure 10: Forest plot for rs2305949 (SEQ ID NO. 3) in KDR, for hypertension in white BEV-treated subjects.
Figure 11: Hypertension frequencies in correlation to rs4444903 (SEQ ID NO. 4) (EGF).
Figure 12: Forest plot for rs4444903 (SEQ ID NO. 4) in EGF and hypertension in *PGx-SP-BEV-White.*
Figure 13: Forest plot for the association of rs11133360 (SEQ ID NO. 5) with PFS in white BEV-treated subjects.

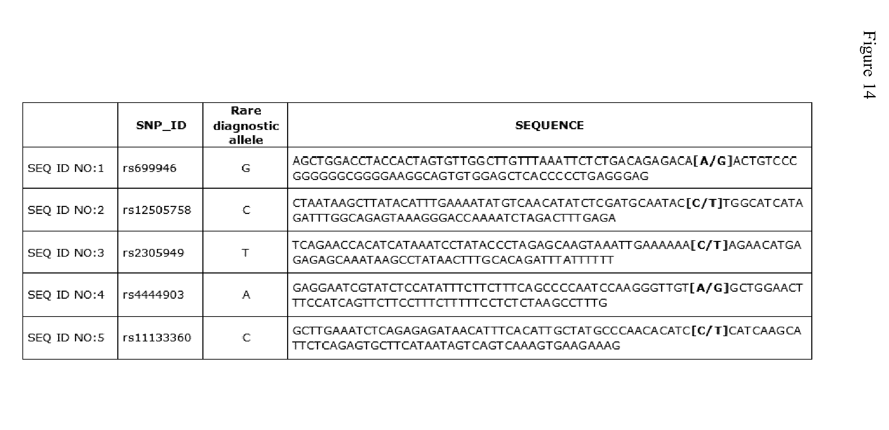

Figure 14: Sequence of SNPs genotyped in the meta-analysis and associated with bevacizumab outcome. SEQ ID NO.1 corresponds to rs699946, wherein position 51 is A or G. SEQ ID NO.2 corresponds to rs12505758, wherein position 51 is C or T. SEQ ID NO.3 corresponds to rs2305949, wherein position 51 is C or T. SEQ ID NO.4 corresponds to rs4444903, wherein position 51 is A or G. SEQ ID NO.5 corresponds to rs11133360, wherein position 51 is C or T.

### Detailed Description of the Embodiments

### 1. Definitions

The term "administering" means the administration of a pharmaceutical composition, such as an angiogenesis inhibitor, to the patient. For example, 2.5 mg/kg of body weight to 15 mg/kg of body weight bevacizumab (Avastin^{®}) can be administered every week, every 2 weeks or every 3 weeks, depending on the type of cancer being treated. Particular dosages include 5 mg/kg, 7.5 mg/kg, 10 mg/kg and 15 mg/kg. Even more particular dosages are 5 mg/kg every 2 weeks, 10 mg/kg every 2 weeks and 15 mg/kg every 3 weeks.

The term "angiogenesis inhibitor" in the context of the present invention refers to all agents that alter angiogenesis (e.g. the process of forming blood vessels) and includes agents that inhibit the angiogenesis, including, but not limited to, tumor angiogenesis. In this context, inhibition can refer to blocking the formation of blood vessels and halting or slowing down the growth of blood vessels. Examples of angiogenesis inhibitors include bevacizumab (also known as Avastin®), pegaptanib, sunitinib, sorafenib and vatalanib. Bevacizumab is a recombinant humanized monoclonal IgG1 antibody that binds to and inhibits the biological activity of human VEGFA in in vitro and in vivo assay system. The term "bevacizumab" encompass all corresponding anti-VEGF antibodies that fulfill the requirements necessary for obtaining a marketing authorization as an identical or biosimilar product in a country or territory selected from the group of countries consisting of the USA, Europe and Japan. In the context of the present invention, an angiogenesis inhibitor includes an antibody that binds essentially the same epitope on VEGF as bevacizumab, more specifically an antibody that binds to the same epitope on VEGF as bevacizumab. An antibody binds "essentially the same epitope" as a reference antibody, when the two antibodies recognize identical or sterically overlapping epitopes. The most widely used and rapid methods for determining whether two epitopes bind to identical or sterically overlapping epitopes are competition assays, which can be configured in all number of different formats, using either labeled antigen or labeled antibody. Usually, the antigen is immobilized on a 96-well plate, and the ability of unlabeled antibodies to block the binding of labeled antibodies is measured using radioactive or enzyme labels.

The term "cancer" refers to the physiological condition in mammals that is typically characterized by unregulated cell proliferation. Examples of cancer include but are not limited to, carcinoma, lymphoma, blastoma, sarcoma and leukemia. More particular examples of such cancers include squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer (including metastic pancreatic cancer), glioblastoma, cervical cancer, ovarian cancer, liver cancer, bladder cancer, hepatoma, breast cancer (including locally advanced, recurrent or metastatic HER-2 negative breast cancer), colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome.

The term "chemotherapeutic agent" or "chemotherapy regimen" includes any active agent that can provide an anticancer therapeutic effect and may be a chemical agent or a biological agent, in particular, that are capable of interfering with cancer or tumor cells. Particular active agents are those that act as anti-neoplastic (chemotoxic or chemostatic) agents which inhibit or prevent the development, maturation or proliferation of malignant cells. Examples of chemotherapeutic agents include alkylating agents such as nitrogen mustards (e.g., mechlorethamine, cyclophosphamide, ifosfamide, melphalan and chlorambucil), nitrosoureas (e.g., carmustine (BCNU), lomustine (CCNU), and semustine (methyl-CCNU)), ethylenimines/ methylmelamines (e.g., thriethylenemelamine (TEM), triethylene, thiophosphoramide (thiotepa), hexamethylmelamine (HMM, altretamine)), alkyl sulfonates (e.g., busulfan), and triazines (e.g., dacarbazine (DTIC)); antimetabolites such as folic acid analogs (e.g., methotrexate, trimetrexate), pyrimidine analogs (e.g., 5-fluorouracil, capecitabine, fluorodeoxyuridine, gemcitabine, cytosine arabinoside (AraC, cytarabine), 5-azacytidine, 2,2'-difluorodeoxycytidine), and purine analogs (e.g., 6-mercaptopurine, 6-thioguanine, azathioprine, 2'-deoxycoformycin (pentostatin), erythrohydroxynonyladenine (EHNA), fludarabine phosphate, and 2-chlorodeoxyadenosine (cladribine, 2-CdA)); antimitotic drugs developed from natural products (e.g., paclitaxel, vinca alkaloids (e.g., vinblastine (VLB), vincristine, and vinorelbine), docetaxel, estramustine, and estramustine phosphate), epipodophylotoxins (.e.g., etoposide, teniposide), antibiotics (.e.g, actimomycin D, daunomycin (rubidomycin), daunorubicon, doxorubicin, epirubicin, mitoxantrone, idarubicin, bleomycins, plicamycin (mithramycin), mitomycinC, actinomycin), enzymes (e.g., L-asparaginase), and biological response modifiers (e.g., interferon-alpha, IL-2, G-CSF, GM-CSF); miscellaneous agents including platinum coordination complexes (e.g., cisplatin, carboplatin, oxaliplatin), anthracenediones (e.g., mitoxantrone), substituted urea (i.e., hydroxyurea), methylhydrazine derivatives (e.g., N-methylhydrazine (MIH), procarbazine), adrenocortical suppressants (e.g., mitotane (o,p'-DDD), aminoglutethimide); hormones and antagonists including adrenocorticosteroid antagonists (.e.g, prednisone and equivalents, dexamethasone, aminoglutethimide), progestins (e.g., hydroxyprogesterone caproate, medroxyprogesterone acetate, megestrol acetate), estrogens (e.g., diethylstilbestrol, ethinyl estradiol and equivalents thereof); antiestrogens (e.g., tamoxifen), androgens (e.g., testosterone propionate, fluoxymesterone and equivalents thereof), antiandrogens (e.g., flutamide, gonadotropin-releasing hormone analogs, leuprolide), non-steroidal antiandrogens (e.g., flutamide), epidermal growth factor inhibitors (e.g., erlotinib, lapatinib, gefitinib) antibodies (e.g., trastuzumab), irinotecan and other agents such as leucovorin. For the treatment of metastatic pancreatic cancer, chemotherapeutic agents for administration with bevacizumab include gemcitabine and erlotinib and combinations thereof (see also the examples herein provided). For the treatment of renal cell cancer, chemotherapeutic agents for administration with bevacizumab include interferon alpha (see also the examples herein provided).

The term "allele" refers to a nucleotide sequence variant of a gene of interest.

The term "genotype" refers to a description of the alleles of a gene contained in an individual or a sample. In the context of this invention, no distinction is made between the genotype of an individual and the genotype of a sample originating from the individual. Although typically a genotype is determined from samples of diploid cells, a genotype can be determined from a sample of haploid cells, such as a sperm cell.

The terms "oligonucleotide" and "polynucleotide" are used interchangeably and refer to a molecule comprised of two or more deoxyribonucleotides or ribonucleotides, preferably more than three. Its exact size will depend on many factors, which in turn depend on the ultimate function or use of the oligonucleotide. An oligonucleotide can be derived synthetically or by cloning. Chimeras of deoxyribonucleotides and ribonucleotides may also be in the scope of the present invention.

The term "polymorphism" refers to the occurrence of two or more genetically determined alternative sequences of a gene in a population. Typically, the first identified allelic form is arbitrarily designated as the reference form and other allelic forms are designated as alternative or variant alleles. The allelic form occurring most frequently in a selected population is sometimes referred to as the wildtype form.

The term a "single nucleotide polymorphism" or "SNP" is a site of one nucleotide that varies between alleles. Single nucleotide polymorphisms may occur at any region of the gene. In some instances the polymorphism can result in a change in protein sequence. The change in protein sequence may affect protein function or not.

The term "hypertension" refers to high blood pressure. The "hypertension associated with a therapy" can be measured with different grades according to the National Cancer Institute's Common Terminology Criteria for Adverse Events (CTCAE v2-3). As the skilled person will appreciate, a patient is more susceptible to developing hypertension, if the patient belongs to a subgroup of patients that has a statistically significant likelihood of developing hypertension as compared to another subgroup of patients. Likewise, a patient is less susceptible to developing hypertension, if the patient belongs to a subgroup of patients that has a statistically significant likelihood of not developing hypertension as compared to another subgroup of patients.

The term "patient" refers to any single animal, more specifically a mammal (including such non-human animals as, for example, dogs, cats, horses, rabbits, zoo animals, cows, pigs, sheep, and non-human primates) for which treatment is desired. Even more specifically, the patient herein is a human. In the context of the present invention, the patient may be a white subject.

The term "subject" herein is any single human subject, including a patient, eligible for treatment who is experiencing or has experienced one or more signs, symptoms, or other indicators of an angiogenic disorder. Intended to be included as a subject are any subjects involved in clinical research trials not showing any clinical sign of disease, or subjects involved in epidemiological studies, or subjects once used as controls. The subject may have been previously treated with an anti-cancer agent, or not so treated. The subject may be naïve to an additional agent(s) being used when the treatment herein is started, *i*.*e*., the subject may not have been previously treated with, for example, an anti-neoplastic agent, a chemotherapeutic agent, a growth inhibitory agent, a cytotoxic agent at "baseline" (*i.e.*, at a set point in time before the administration of a first dose of an anti-cancer in the treatment method herein, such as the day of screening the subject before treatment is commenced). Such "naïve" subjects are generally considered to be candidates for treatment with such additional agent(s).

The term "a patient suffering from" refers to a patient showing clinical signs in respect to a certain malignant disease, such as cancer, a disease involving physiological and pathological angiogenesis and/or tumorous disease.

As used herein, "therapy" or "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis.

The term "overall survival" refers to the length of time during and after treatment the patient survives.

The term "progression-free survival" refers to the length of time during and after treatment during which, according to the assessment of the treating physician or investigator, the patient's disease does not become worse, *i.e.*, does not progress.

The term "pharmaceutical composition" refers to a sterile preparation that is in such form as to permit the biological activity of the medicament to be effective, and which contains no additional components that are unacceptably toxic to a subject to which the formulation would be administered.

### 2. Detailed Embodiments

In the present invention, variations in the KDR and EGF genes were surprisingly identified as markers/predictors for susceptibility to developing hypertension associated with treatment by an angiogenesis inhibitor. The terms "marker" and "predictor" can be used interchangeably and refer to specific allele variants of genes. The variation or marker may also be referred to as a single nucleotide polymorphism (SNP).

In accordance with the methods of the present invention, a meta-analysis of SNPs was conducted using the samples derived from five Phase II and Phase III trials with bevacizumab, i.e. NO16966 (advanced primary colorectal cancer, *see,* Saltz et al., 2008, J. Clin. Oncol. 26:2013-2019 and Hurwitz et al., 2004, N. Engl. J. Med. 350:2335-2342), AVITA (pancreatic cancer, *see*, Van Cutsem, J. Clin. Oncol. 2009 27:2231-2237), AVAiL (non-small cell lung cancer, *see*, Reck et al., J. Clin. Oncol. 2009 27:1227), AVOREN (renal cancer, *see*, Escudier et al., J. Clin. Oncol. 2010 28:2144) and AVADO (breast cancer, *see*, Miles, J. Clin. Oncol. 2010 28:3239).

As shown in the examples, ten SNPs were associated with bevacizumab-induced hypertension (p<0.05), but none of these surpassed the threshold for multiple testing (p<0.0003). The two SNPs showing the strongest association (p<0.01) were: rs2305949 (SEQ ID NO. 3) in KDR (allelic OR 0.93, 95% CI 0.88-0.98, p=0.0067) and rs4444903 (SEQ ID NO. 4) in EGF (allelic OR 1.06, 95% CI 1.02-1.11, p=0.0052). For rs2305949 (SEQ ID NO. 3) (KDR), CC carriers (wildtype) showed a higher frequency of developing hypertension when treated with BEV, while patients treated with placebo did show a rather opposite result, showing highest hypetrtension frequency in patients carrying TT. For rs4444903 (SEQ ID NO. 4) (EGF), the heterozygote GA carriers showed the highest frequency of hypertension, an effect that was not seen in the placebo treated patients, as no differences were seen between different genotypes. Interestingly, rs2305949 (SEQ ID NO. 3) and rs4444903 (SEQ ID NO. 4) were closely linked to amino acid changes occurring on position 273 and 708 of KDR and EGF, suggesting that these changes may functionally affect both genes and thereby contribute to hypertension.

Accordingly, the present invention provides an in vitro method of determining susceptibility of a patient to developing hypertension associated with a therapy by an angiogenesis inhibitor comprising bevacizumab or an antibody that binds essentially the same epitope on VEGF as bevacizumab, said method comprising:
(a) determining in a sample derived from a patient suffering from cancer the genotype at polymorphism rs2305949 (SEQ ID NO. 3), and
(b) identifying a patient as more or less susceptible to developing hypertension associated with a therapy by an angiogenesis inhibitor comprising bevacizumab or an antibody that binds essentially the same epitope on VEGF as bevacizumab based on said genotype, wherein the presence of CC genotype at polymorphism rs2305949 (SEQ ID NO. 3) indicates that said patient is more susceptible to developing hypertension than a patient having a genotype of CT or TT at polymorphism rs2305949 (SEQ ID NO. 3), or the presence of CT or TT genotype at polymorphism rs2305949 (SEQ ID NO. 3) indicates that said patient is less susceptible to developing hypertension than a patient having a genotype of CC at polymorphism rs2305949 (SEQ ID NO. 3). In an embodiment, cancer is selected from the group consisting of colorectal cancer, glioblastoma, renal cancer, ovarian cancer, breast cancer, pancreatic cancer, gastric cancer and lung cancer, more specifically colorectal cancer, renal cancer, breast cancer, pancreatic cancer and lung cancer, even more specifically colorectal cancer, renal cancer and lung cancer.

The present invention further provides a pharmaceutical composition comprising an angiogenesis inhibitor that comprises bevacizumab or an antibody that binds essentially the same epitope on VEGF as bevacizumab, for the treatment of a patient in need thereof, wherein said patient has been determined to be less susceptible to developing hypertension associated with a therapy by the angiogenesis inhibitor by an in vitro method comprising:
(a) determining in a sample derived from a patient suffering from cancer the genotype at polymorphism rs2305949 (SEQ ID NO. 3), and
(b) identifying a patient as more or less susceptible to developing hypertension associated with a therapy by an angiogenesis inhibitor comprising bevacizumab or an antibody that binds essentially the same epitope on VEGF as bevacizumab based on said genotype, wherein the presence of CC genotype at polymorphism rs2305949 (SEQ ID NO. 3) indicates that said patient is more susceptible to developing hypertension than a patient having a genotype of CT or TT at polymorphism rs2305949 (SEQ ID NO. 3), or the presence of CT or TT genotype at polymorphism rs2305949 (SEQ ID NO. 3) indicates that said patient is less susceptible to developing hypertension than a patient having a genotype of CC at polymorphism rs2305949 (SEQ ID NO. 3). In an embodiment, cancer is selected from the group consisting of colorectal cancer, glioblastoma, renal cancer, ovarian cancer, breast cancer, pancreatic cancer, gastric cancer and lung cancer, more specifically colorectal cancer, renal cancer, breast cancer, pancreatic cancer and lung cancer, even more specifically colorectal cancer, renal cancer and lung cancer.

The present invention further provides a method of reducing the risk of developing hypertension associated with a therapy by an angiogenesis inhibitor comprising bevacizumab or an antibody that binds essentially the same epitope on VEGF as bevacizumab, said method comprising:
(a) determining in a sample derived from a patient suffering from cancer the genotype at polymorphism rs2305949 (SEQ ID NO. 3);
(b) identifying a patient as less susceptible to developing hypertensin associated with a therapy by an angiogenesis inhibitor comprising bevacizumab or an antibody that binds essentially the same epitope on VEGF as bevacizumab, wherein the presence of CT or TT genotype at polymorphism rs2305949 (SEQ ID NO. 3) indicates that said patient is less susceptible to developing hypertension than a patient having a genotype of CC at polymorphism rs2305949 (SEQ ID NO. 3); and
(c) administering said angiogenesis inhibitor to a patient with the CT or TT genotype at polymorphism rs2305949 (SEQ ID NO. 3) identified as less susceptible to developing hypertension in accordance with (b). In an embodiment, cancer is selected from the group consisting of colorectal cancer, glioblastoma, renal cancer, ovarian cancer, breast cancer, pancreatic cancer, gastric cancer and lung cancer, more specifically colorectal cancer, renal cancer, breast cancer, pancreatic cancer and lung cancer, even more specifically colorectal cancer, renal cancer and lung cancer.

The present invention also provides an in vitro method of determining susceptibility of a patient to developing hypertension associated with a therapy by an angiogenesis inhibitor comprising bevacizumab or an antibody that binds essentially the same epitope on VEGF as bevacizumab, said method comprising:
(a) determining in a sample derived from a patient suffering from cancer the genotype at polymorphism rs4444903 (SEQ ID NO. 4), and
(b) identifying a patient as more or less susceptible to developing hypertension associated with a therapy by an angiogenesis inhibitor comprising bevacizumab or an antibody that binds essentially the same epitope on VEGF as bevacizumab based on said genotype, wherein the presence of GA genotype at polymorphism rs4444903 (SEQ ID NO. 4) indicates that said patient is more susceptible to developing hypertension than a patient having a genotype of GG or AA at polymorphism rs4444903 (SEQ ID NO. 4), or the presence of GG or AA genotype at polymorphism rs4444903 (SEQ ID NO. 4) indicates that said patient is less susceptible to developing hypertension than a patient having a genotype of GA at polymorphism rs4444903 (SEQ ID NO. 4). In an embodiment, cancer is selected from the group consisting of colorectal cancer, glioblastoma, renal cancer, ovarian cancer, breast cancer, pancreatic cancer, gastric cancer and lung cancer, more specifically colorectal cancer, renal cancer, breast cancer, pancreatic cancer and lung cancer, even more specifically colorectal cancer, renal cancer and breast cancer.

The present invention further provides a pharmaceutical composition comprising an angiogenesis inhibitor that comprises bevacizumab or an antibody that binds essentially the same epitope on VEGF as bevacizumab, for the treatment of a patient in need thereof, wherein said patient has been determined to be less susceptible to developing hypertension associated with a therapy by the angiogenesis inhibitor by an in vitro method comprising:
(a) determining in a sample derived from a patient suffering from cancer the genotype at polymorphism rs4444903 (SEQ ID NO. 4), and
(b) identifying a patient as more or less susceptible to developing hypertension associated with a therapy by an angiogenesis inhibitor comprising bevacizumab or an antibody that binds essentially the same epitope on VEGF as bevacizumab based on said genotype, wherein the presence of GA genotype at polymorphism rs4444903 (SEQ ID NO. 4) indicates that said patient is more susceptible to developing hypertension than a patient having a genotype of GG or AA at polymorphism rs4444903 (SEQ ID NO. 4), or the presence of GG or AA genotype at polymorphism rs4444903 (SEQ ID NO. 4) indicates that said patient is less susceptible to developing hypertension than a patient having a genotype of GA at polymorphism rs4444903 (SEQ ID NO. 4). In an embodiment, cancer is selected from the group consisting of colorectal cancer, glioblastoma, renal cancer, ovarian cancer, breast cancer, pancreatic cancer, gastric cancer and lung cancer, more specifically colorectal cancer, renal cancer, breast cancer, pancreatic cancer and lung cancer, even more specifically colorectal cancer, renal cancer and breast cancer.

The present invention further provides a method of reducing the risk of developing hypertension associated with a therapy by an angiogenesis inhibitor comprising bevacizumab or an antibody that binds essentially the same epitope on VEGF as bevacizumab, said method comprising:
(a) determining in a sample derived from a patient suffering from cancer the genotype at polymorphism rs4444903 (SEQ ID NO. 4);
(b) identifying a patient as less susceptible to developing hypertensin associated with a therapy by an angiogenesis inhibitor comprising bevacizumab or an antibody that binds essentially the same epitope on VEGF as bevacizumab, wherein the presence of GG or AA genotype at polymorphism rs4444903 (SEQ ID NO. 4) indicates that said patient is less susceptible to developing hypertension than a patient having a genotype of GA at polymorphism rs4444903 (SEQ ID NO. 4); and
(c) administering said angiogenesis inhibitor to a patient with the GG or AA genotype at polymorphism rs4444903 (SEQ ID NO. 4) identified as less susceptible to developing hypertension in accordance with (b). In an embodiment, cancer is selected from the group consisting of colorectal cancer, glioblastoma, renal cancer, ovarian cancer, breast cancer, pancreatic cancer, gastric cancer and lung cancer, more specifically colorectal cancer, renal cancer, breast cancer, pancreatic cancer and lung cancer, even more specifically colorectal cancer, renal cancer and lung cancer.

In an embodiment, the angiogenesis inhibitor is administered as a co-treatment with a chemotherapeutic agent or chemotherapy regimen. In a further embodiment, the angiogenesis inhibitor is administered with one or more agents selected from the group consisting of taxanes such as docetaxel and paclitaxel, interferon alpha, 5-fluorouracil, leucovorin, irinotecan, gemcitabine-erlotinib and platinum-based chemotherapeutic agents such as carboplatin, cisplatin and oxaliplatin. Further, the angiogenesis inhibitor may be administered as a co-treatment with radiotherapy.

In the context of the present invention, the sample is a biological sample and may be a blood and/or tissue sample. In an embodiment, the sample is a blood sample, more specifically a peripheral blood sample. In the context of the present invention, the sample is a DNA sample. The DNA sample may be germline DNA or somatic DNA, more specifically germline DNA.

In one embodiment, the genotype is determined by means of MALDI-TOF mass spectrometry. In addition to the detailed description of the detection of SNPs below, the following reference provides guidance for MALDI-TOF mass spectrometry-based SNP genotyping, e.g. Storm et al., Methods Mol. Biol. 212:241-62, 2003.

### 3. Detection of Nucleic Acid Polymorphisms

Detection techniques for evaluating nucleic acids for the presence of a SNP involve procedures well known in the field of molecular genetics. Many, but not all, of the methods involve amplification of nucleic acids. Ample guidance for performing amplification is provided in the art. Exemplary references include manuals such as PCR Technology: Principles and Applications for DNA Amplification (ed. H. A. Erlich, Freeman Press, NY, N.Y., 1992); PCR Protocols: A Guide to Methods and Applications (eds. Innis, et al., Academic Press, San Diego, Calif., 1990); Current Protocols in Molecular Biology, Ausubel, 1994-1999, including supplemental updates through April 2004; Sambrook & Russell, Molecular Cloning, A Laboratory Manual (3rd Ed, 2001). General methods for detection of single nucleotide polymorphisms are disclosed in Single Nucleotide Polymorphisms: Methods and Protocols, Pui-Yan Kwok, ed., 2003, Humana Press.

Although the methods typically employ PCR steps, other amplification protocols may also be used. Suitable amplification methods include ligase chain reaction (see, e.g., Wu & Wallace, Genomics 4:560-569, 1988); strand displacement assay (see, e.g. Walker et al., Proc. Natl. Acad. Sci. USA 89:392-396, 1992; U.S. Pat. No. 5,455,166); and several transcription-based amplification systems, including the methods described in U.S. Pat. Nos. 5,437,990; 5,409,818; and 5,399,491; the transcription amplification system (TAS) (Kwoh et al., Proc. Natl. Acad. Sci. USA 86:1173-1177, 1989); and self-sustained sequence replication (3SR) (Guatelli et al., Proc. Natl. Acad. Sci. USA 87:1874-1878, 1990; WO 92/08800). Alternatively, methods that amplify the probe to detectable levels can be used, such as Qβ-replicase amplification (Kramer & Lizardi, Nature 339:401-402, 1989; Lomeli et al., Clin. Chem. 35:1826-1831, 1989). A review of known amplification methods is provided, for example, by Abramson and Myers in Current Opinion in Biotechnology 4:41-47, 1993.

Detection of the genotype, haplotype, SNP, microsatellite or other polymorphism of an individual can be performed using oligonucleotide primers and/or probes. Oligonucleotides can be prepared by any suitable method, usually chemical synthesis. Oligonucleotides can be synthesized using commercially available reagents and instruments. Alternatively, they can be purchased through commercial sources. Methods of synthesizing oligonucleotides are well known in the art (see, e.g, Narang et al., Meth. Enzymol. 68:90-99, 1979; Brown et al., Meth. Enzymol. 68:109-151, 1979; Beaucage et al., Tetrahedron Lett. 22:1859-1862, 1981; and the solid support method of U.S. Pat. No. 4,458,066). In addition, modifications to the above-described methods of synthesis may be used to desirably impact enzyme behavior with respect to the synthesized oligonucleotides. For example, incorporation of modified phosphodiester linkages (e.g., phosphorothioate, methylphosphonates, phosphoamidate, or boranophosphate) or linkages other than a phosphorous acid derivative into an oligonucleotide may be used to prevent cleavage at a selected site. In addition, the use of 2'-amino modified sugars tends to favor displacement over digestion of the oligonucleotide when hybridized to a nucleic acid that is also the template for synthesis of a new nucleic acid strand.

The genotype of an individual can be determined using many detection methods that are well known in the art. Most assays entail one of several general protocols: hybridization using allele-specific oligonucleotides, primer extension, allele-specific ligation, sequencing, or electrophoretic separation techniques, e.g., single-stranded conformational polymorphism (SSCP) and heteroduplex analysis. Exemplary assays include 5'-nuclease assays, template-directed dye-terminator incorporation, molecular beacon allele-specific oligonucleotide assays, single-base extension assays, and SNP scoring by real-time pyrophosphate sequences. Analysis of amplified sequences can be performed using various technologies such as microchips, fluorescence polarization assays, and MALDI-TOF (matrix assisted laser desorption ionization-time of flight) mass spectrometry. Two methods that can also be used are assays based on invasive cleavage with Flap nucleases and methodologies employing padlock probes.

Determination of the presence or absence of a particular allele is generally performed by analyzing a nucleic acid sample that is obtained from the individual to be analyzed. Often, the nucleic acid sample comprises genomic DNA. The genomic DNA is typically obtained from blood samples, but may also be obtained from other cells or tissues.

It is also possible to analyze RNA samples for the presence of polymorphic alleles. For example, mRNA can be used to determine the genotype of an individual at one or more polymorphic sites. In this case, the nucleic acid sample is obtained from cells in which the target nucleic acid is expressed, e.g., adipocytes. Such an analysis can be performed by first reverse-transcribing the target RNA using, for example, a viral reverse transcriptase, and then amplifying the resulting cDNA; or using a combined high-temperature reverse-transcription-polymerase chain reaction (RT-PCR), as described in U.S. Pat. Nos. 5,310,652; 5,322,770; 5,561,058; 5,641,864; and 5,693,517.

Frequently used methodologies for analysis of nucleic acid samples to detect SNPs are briefly described. However, any method known in the art can be used in the invention to detect the presence of single nucleotide substitutions.

### a. Allele-Specific Hybridization

This technique, also commonly referred to as allele specific oligonucleotide hybridization (ASO) (e.g., Stoneking et al., Am. J. Hum. Genet. 48:70-382, 1991; Saiki et al., Nature 324, 163-166, 1986; EP 235,726; and WO 89/11548), relies on distinguishing between two DNA molecules differing by one base by hybridizing an oligonucleotide probe that is specific for one of the variants to an amplified product obtained from amplifying the nucleic acid sample. This method typically employs short oligonucleotides, e.g. 15-20 bases in length. The probes are designed to differentially hybridize to one variant versus another. Principles and guidance for designing such probe is available in the art, e.g. in the references cited herein. Hybridization conditions should be sufficiently stringent that there is a significant difference in hybridization intensity between alleles, and producing an essentially binary response, whereby a probe hybridizes to only one of the alleles. Some probes are designed to hybridize to a segment of target DNA such that the polymorphic site aligns with a central position (e.g., in a 15-base oligonucleotide at the 7 position; in a 16-based oligonucleotide at either the 8 or 9 position) of the probe, but this design is not required.

The amount and/or presence of an allele is determined by measuring the amount of allele-specific oligonucleotide that is hybridized to the sample. Typically, the oligonucleotide is labeled with a label such as a fluorescent label. For example, an allele-specific oligonucleotide is applied to immobilized oligonucleotides representing SNP sequences. After stringent hybridization and washing conditions, fluorescence intensity is measured for each SNP oligonucleotide.

In one embodiment, the nucleotide present at the polymorphic site is identified by hybridization under sequence-specific hybridization conditions with an oligonucleotide probe or primer exactly complementary to one of the polymorphic alleles in a region encompassing the polymorphic site. The probe or primer hybridizing sequence and sequence-specific hybridization conditions are selected such that a single mismatch at the polymorphic site destabilizes the hybridization duplex sufficiently so that it is effectively not formed. Thus, under sequence-specific hybridization conditions, stable duplexes will form only between the probe or primer and the exactly complementary allelic sequence. Thus, oligonucleotides from about 10 to about 35 nucleotides in length, usually from about 15 to about 35 nucleotides in length, which are exactly complementary to an allele sequence in a region which encompasses the polymorphic site are within the scope of the invention.

In an alternative embodiment, the nucleotide present at the polymorphic site is identified by hybridization under sufficiently stringent hybridization conditions with an oligonucleotide substantially complementary to one of the SNP alleles in a region encompassing the polymorphic site, and exactly complementary to the allele at the polymorphic site. Because mismatches which occur at non-polymorphic sites are mismatches with both allele sequences, the difference in the number of mismatches in a duplex formed with the target allele sequence and in a duplex formed with the corresponding non-target allele sequence is the same as when an oligonucleotide exactly complementary to the target allele sequence is used. In this embodiment, the hybridization conditions are relaxed sufficiently to allow the formation of stable duplexes with the target sequence, while maintaining sufficient stringency to preclude the formation of stable duplexes with non-target sequences. Under such sufficiently stringent hybridization conditions, stable duplexes will form only between the probe or primer and the target allele. Thus, oligonucleotides from about 10 to about 35 nucleotides in length, usually from about 15 to about 35 nucleotides in length, which are substantially complementary to an allele sequence in a region which encompasses the polymorphic site, and are exactly complementary to the allele sequence at the polymorphic site, are within the scope of the invention.

The use of substantially, rather than exactly, complementary oligonucleotides may be desirable in assay formats in which optimization of hybridization conditions is limited. For example, in a typical multi-target immobilized-oligonucleotide assay format, probes or primers for each target are immobilized on a single solid support. Hybridizations are carried out simultaneously by contacting the solid support with a solution containing target DNA. As all hybridizations are carried out under identical conditions, the hybridization conditions cannot be separately optimized for each probe or primer. The incorporation of mismatches into a probe or primer can be used to adjust duplex stability when the assay format precludes adjusting the hybridization conditions. The effect of a particular introduced mismatch on duplex stability is well known, and the duplex stability can be routinely both estimated and empirically determined, as described above. Suitable hybridization conditions, which depend on the exact size and sequence of the probe or primer, can be selected empirically using the guidance provided herein and well known in the art. The use of oligonucleotide probes or primers to detect single base pair differences in sequence is described in, for example, Conner et al., 1983, Proc. Natl. Acad. Sci. USA 80:278-282, and U.S. Pat. Nos. 5,468,613 and 5,604,099, each incorporated herein by reference.

The proportional change in stability between a perfectly matched and a single-base mismatched hybridization duplex depends on the length of the hybridized oligonucleotides. Duplexes formed with shorter probe sequences are destabilized proportionally more by the presence of a mismatch. Oligonucleotides between about 15 and about 35 nucleotides in length are often used for sequence-specific detection. Furthermore, because the ends of a hybridized oligonucleotide undergo continuous random dissociation and re-annealing due to thermal energy, a mismatch at either end destabilizes the hybridization duplex less than a mismatch occurring internally. For discrimination of a single base pair change in target sequence, the probe sequence is selected which hybridizes to the target sequence such that the polymorphic site occurs in the interior region of the probe.

The above criteria for selecting a probe sequence that hybridizes to a specific allele apply to the hybridizing region of the probe, i.e., that part of the probe which is involved in hybridization with the target sequence. A probe may be bound to an additional nucleic acid sequence, such as a poly-T tail used to immobilize the probe, without significantly altering the hybridization characteristics of the probe. One of skill in the art will recognize that for use in the present methods, a probe bound to an additional nucleic acid sequence which is not complementary to the target sequence and, thus, is not involved in the hybridization, is essentially equivalent to the unbound probe.

Suitable assay formats for detecting hybrids formed between probes and target nucleic acid sequences in a sample are known in the art and include the immobilized target (dot-blot) format and immobilized probe (reverse dot-blot or line-blot) assay formats. Dot blot and reverse dot blot assay formats are described in U.S. Pat. Nos. 5,310,893; 5,451,512; 5,468,613; and 5,604,099; each incorporated herein by reference.

In a dot-blot format, amplified target DNA is immobilized on a solid support, such as a nylon membrane. The membrane-target complex is incubated with labeled probe under suitable hybridization conditions, unhybridized probe is removed by washing under suitably stringent conditions, and the membrane is monitored for the presence of bound probe.

In the reverse dot-blot (or line-blot) format, the probes are immobilized on a solid support, such as a nylon membrane or a microtiter plate. The target DNA is labeled, typically during amplification by the incorporation of labeled primers. One or both of the primers can be labeled. The membrane-probe complex is incubated with the labeled amplified target DNA under suitable hybridization conditions, unhybridized target DNA is removed by washing under suitably stringent conditions, and the membrane is monitored for the presence of bound target DNA. A reverse line-blot detection assay is described in the example.

An allele-specific probe that is specific for one of the polymorphism variants is often used in conjunction with the allele-specific probe for the other polymorphism variant. In some embodiments, the probes are immobilized on a solid support and the target sequence in an individual is analyzed using both probes simultaneously. Examples of nucleic acid arrays are described by WO 95/11995. The same array or a different array can be used for analysis of characterized polymorphisms. WO 95/11995 also describes subarrays that are optimized for detection of variant forms of a pre-characterized polymorphism. Such a subarray can be used in detecting the presence of the polymorphisms described herein.

### b. Allele-Specific Primers

Polymorphisms are also commonly detected using allele-specific amplification or primer extension methods. These reactions typically involve use of primers that are designed to specifically target a polymorphism via a mismatch at the 3'-end of a primer. The presence of a mismatch effects the ability of a polymerase to extend a primer when the polymerase lacks error-correcting activity. For example, to detect an allele sequence using an allele-specific amplification- or extension-based method, a primer complementary to one allele of a polymorphism is designed such that the 3'-terminal nucleotide hybridizes at the polymorphic position. The presence of the particular allele can be determined by the ability of the primer to initiate extension. If the 3'-terminus is mismatched, the extension is impeded.

In some embodiments, the primer is used in conjunction with a second primer in an amplification reaction. The second primer hybridizes at a site unrelated to the polymorphic position. Amplification proceeds from the two primers leading to a detectable product signifying the particular allelic form is present. Allele-specific amplification- or extension-based methods are described in, for example, WO 93/22456; U.S. Pat. Nos. 5,137,806; 5,595,890; 5,639,611; and U.S. Pat. No. 4,851,331.

Using allele-specific amplification-based genotyping, identification of the alleles requires only detection of the presence or absence of amplified target sequences. Methods for the detection of amplified target sequences are well known in the art. For example, gel electrophoresis and probe hybridization assays described are often used to detect the presence of nucleic acids.

In an alternative probe-less method, the amplified nucleic acid is detected by monitoring the increase in the total amount of double-stranded DNA in the reaction mixture, is described, e.g. in U.S. Pat. No. 5,994,056; and European Patent Publication Nos. 487,218 and 512,334. The detection of double-stranded target DNA relies on the increased fluorescence various DNA-binding dyes, e.g., SYBR Green, exhibit when bound to double-stranded DNA.

As appreciated by one in the art, allele-specific amplification methods can be performed in reaction that employ multiple allele-specific primers to target particular alleles. Primers for such multiplex applications are generally labeled with distinguishable labels or are selected such that the amplification products produced from the alleles are distinguishable by size. Thus, for example, both alleles in a single sample can be identified using a single amplification by gel analysis of the amplification product.

As in the case of allele-specific probes, an allele-specific oligonucleotide primer may be exactly complementary to one of the polymorphic alleles in the hybridizing region or may have some mismatches at positions other than the 3'-terminus of the oligonucleotide, which mismatches occur at non-polymorphic sites in both allele sequences.

### c. Detectable Probes

### i) 5'-Nuclease Assay Probes

Genotyping can also be performed using a "TaqMan®" or " 5'-nuclease assay", as described in U.S. Pat. Nos. 5,210,015; 5,487,972; and 5,804,375; and Holland et al., 1988, Proc. Natl. Acad. Sci. USA 88:7276-7280. In the TaqMan® assay, labeled detection probes that hybridize within the amplified region are added during the amplification reaction. The probes are modified so as to prevent the probes from acting as primers for DNA synthesis. The amplification is performed using a DNA polymerase having 5'- to 3'-exonuclease activity. During each synthesis step of the amplification, any probe which hybridizes to the target nucleic acid downstream from the primer being extended is degraded by the 5'- to 3'-exonuclease activity of the DNA polymerase. Thus, the synthesis of a new target strand also results in the degradation of a probe, and the accumulation of degradation product provides a measure of the synthesis of target sequences.

The hybridization probe can be an allele-specific probe that discriminates between the SNP alleles. Alternatively, the method can be performed using an allele-specific primer and a labeled probe that binds to amplified product.

Any method suitable for detecting degradation product can be used in a 5'-nuclease assay. Often, the detection probe is labeled with two fluorescent dyes, one of which is capable of quenching the fluorescence of the other dye. The dyes are attached to the probe, usually one attached to the 5'-terminus and the other is attached to an internal site, such that quenching occurs when the probe is in an unhybridized state and such that cleavage of the probe by the 5'- to 3'-exonuclease activity of the DNA polymerase occurs in between the two dyes. Amplification results in cleavage of the probe between the dyes with a concomitant elimination of quenching and an increase in the fluorescence observable from the initially quenched dye. The accumulation of degradation product is monitored by measuring the increase in reaction fluorescence. U.S. Pat. Nos. 5,491,063 and 5,571,673, both incorporated herein by reference, describe alternative methods for detecting the degradation of probe which occurs concomitant with amplification.

### ii) Secondary Structure Probes

Probes detectable upon a secondary structural change are also suitable for detection of a polymorphism, including SNPs. Exemplified secondary structure or stem-loop structure probes include molecular beacons or Scorpion® primer/probes. Molecular beacon probes are single-stranded oligonucleic acid probes that can form a hairpin structure in which a fluorophore and a quencher are usually placed on the opposite ends of the oligonucleotide. At either end of the probe short complementary sequences allow for the formation of an intramolecular stem, which enables the fluorophore and the quencher to come into close proximity. The loop portion of the molecular beacon is complementary to a target nucleic acid of interest. Binding of this probe to its target nucleic acid of interest forms a hybrid that forces the stem apart. This causes a conformation change that moves the fluorophore and the quencher away from each other and leads to a more intense fluorescent signal. Molecular beacon probes are, however, highly sensitive to small sequence variation in the probe target (Tyagi S. and Kramer F. R., Nature Biotechnology, Vol. 14, pages 303-308 (1996); Tyagi et al., Nature Biotechnology, Vol. 16, pages 49-53(1998); Piatek et al., Nature Biotechnology, Vol. 16, pages 359-363 (1998); Marras S. et al., Genetic Analysis: Biomolecular Engineering, Vol. 14, pages 151-156 (1999); Tpp I. et al, BioTechniqates, Vol 28, pages 732-738 (2000)). A Scorpion® primer/probe comprises a stem-loop structure probe covalently linked to a primer.

### d. DNA Sequencing and Single Base Extensions

SNPs can also be detected by direct sequencing. Methods include e.g. dideoxy sequencing-based methods and other methods such as Maxam and Gilbert sequence (see, e.g. Sambrook and Russell, supra).

Other detection methods include Pyrosequencing™ of oligonucleotide-length products. Such methods often employ amplification techniques such as PCR. For example, in pyrosequencing, a sequencing primer is hybridized to a single stranded, PCR-amplified, DNA template; and incubated with the enzymes, DNA polymerase, ATP sulfurylase, luciferase and apyrase, and the substrates, adenosine 5' phosphosulfate (APS) and luciferin. The first of four deoxynucleotide triphosphates (dNTP) is added to the reaction. DNA polymerase catalyzes the incorporation of the deoxynucleotide triphosphate into the DNA strand, if it is complementary to the base in the template strand. Each incorporation event is accompanied by release of pyrophosphate (PPi) in a quantity equimolar to the amount of incorporated nucleotide. ATP sulfurylase quantitatively converts PPi to ATP in the presence of adenosine 5' phosphosulfate. This ATP drives the luciferase-mediated conversion of luciferin to oxyluciferin that generates visible light in amounts that are proportional to the amount of ATP. The light produced in the luciferase-catalyzed reaction is detected by a charge coupled device (CCD) camera and seen as a peak in a Pyrogram™. Each light signal is proportional to the number of nucleotides incorporated. Apyrase, a nucleotide degrading enzyme, continuously degrades unincorporated dNTPs and excess ATP. When degradation is complete, another dNTP is added.

Another similar method for characterizing SNPs does not require use of a complete PCR, but typically uses only the extension of a primer by a single, fluorescence-labeled dideoxyribonucleic acid molecule (ddNTP) that is complementary to the nucleotide to be investigated. The nucleotide at the polymorphic site can be identified via detection of a primer that has been extended by one base and is fluorescently labeled (e.g., Kobayashi et al, Mol. Cell. Probes, 9:175-182, 1995).

### e. Electrophoresis

Amplification products generated using the polymerase chain reaction can be analyzed by the use of denaturing gradient gel electrophoresis. Different alleles can be identified based on the different sequence-dependent melting properties and electrophoretic migration of DNA in solution (see, e.g. Erlich, ed., PCR Technology, Principles and Applications for DNA Amplification, W. H. Freeman and Co, New York, 1992, Chapter 7).

Distinguishing of microsatellite polymorphisms can be done using capillary electrophoresis. Capillary electrophoresis conveniently allows identification of the number of repeats in a particular microsatellite allele. The application of capillary electrophoresis to the analysis of DNA polymorphisms is well known to those in the art (see, for example, Szantai, et al, J Chromatogr A. (2005) 1079(1-2):41-9; Bjorheim and Ekstrom, Electrophoresis (2005) 26(13):2520-30 and Mitchelson, Mol Biotechnol. (2003) 24(1):41-68).

### f. Single-Strand Conformation Polymorphism Analysis

Alleles of target sequences can be differentiated using single-strand conformation polymorphism analysis, which identifies base differences by alteration in electrophoretic migration of single stranded PCR products, as described, e.g, in Orita et al., Proc. Nat. Acad. Sci. 86, 2766-2770 (1989). Amplified PCR products can be generated as described above, and heated or otherwise denatured, to form single stranded amplification products. Single-stranded nucleic acids may refold or form secondary structures which are partially dependent on the base sequence. The different electrophoretic mobilities of single-stranded amplification products can be related to base-sequence difference between alleles of target

SNP detection methods often employ labeled oligonucleotides. Oligonucleotides can be labeled by incorporating a label detectable by spectroscopic, photochemical, biochemical, immunochemical, or chemical means. Useful labels include fluorescent dyes, radioactive labels, e.g. 32P, electron-dense reagents, enzyme, such as peroxidase or alkaline phosphatase, biotin, or haptens and proteins for which antisera or monoclonal antibodies are available. Labeling techniques are well known in the art (see, e.g. Current Protocols in Molecular Biology, supra; Sambrook & Russell, supra).

### 4. Methods of Treatment

Dosages of with bevacizumab (Avastin^{®}) for treatments of specific cancers, according to the EMEA, are as follows. For metastatic carcinoma of the colon or rectum (mCRC) recommended dosages are 5 mg/kg or 10 mg/kg of body weight given once every 2 weeks or 7.5 mg/kg or 15 mg/kg of body weight given once every 3 weeks, for metastatic breast cancer (mBC) recommended dosages are 10 mg/kg of body weight given once every 2 weeks or 15 mg/kg of body weight given once every 3 weeks as an intravenous infusion, and for non-small cell lung cancer (NSCLC) recommended dosages are 7.5 mg/kg or 15 mg/kg of body weight given once every 3 weeks as an intravenous infusion. Clinical benefit in NSCLC patients has been demonstrated with both 7.5 mg/kg and 15 mg/kg doses. For details refer to section 5.1 *Pharmacodynamic Properties*, *Non*-*small cell lung cancer (NSCLC).* For advanced and/or metastatic Renal Cell Cancer (mRCC) preferred dosages are 10 mg/kg of body weight given once every 2 weeks as an intravenous infusion(in addition to platinum-based chemotherapy for up to 6 cycles of treatment followed by bevacizumab (Avastin^{®}) as a single agent until disease progression). For gliablastoma a particular dosage is 10 mg/kg every 2 weeks.

In the context of the present invention, the angiogenesis inhibitor may be administered in addition to or as a co-therapy or a co-treatment with one or more chemotherapeutic agents administered as part of standard chemotherapy regimen as known in the art. Examples of agents included in such standard chemotherapy regimens include 5-fluorouracil, leucovorin, irinotecan, gemcitabine, erlotinib, capecitabine, taxanes, such as docetaxel and paclitaxel, interferon alpha, vinorelbine, and platinum-based chemotherapeutic agents, such as paclitaxel, carboplatin, cisplatin and oxaliplatin. Examples of co-treatments for metastatic pancreatic cancer include gemcitabine-erlotinib plus bevacizumab at a dosage of 5mg/kg or 10 mg/kg of body weight given once every two weeks or 7.5 mg/kg or 15 mg/kg of body weight given once every three weeks. Examples of co-treatments for renal cell cancer include interferon alpha plus bevacizumab at a dosage of or 10 mg/kg of body weight given once every two weeks. Further, a patient may be co-treated with a combination of irinotecan, 5-fluorouracil, leucovorin, also referred to as IFL, as, for example, a bolus-IFL, with a combination of oxaliplatin, leucovorin, and 5-fluorouracil, also referred to a FOLFOX4 regimen, or with a combination of capecitabine and oxaliplatin, also referred to as XELOX. Accordingly, in a further embodiment of the invention, the patient suffering from a malignant disease or a disease involving physiological and pathological angiogenesis is being treated with one or more chemotherapeutic agents such as 5-fluorouracil, leucovorin, irinotecan, gemcitabine-erlotinib, capecitabine and/or platinum-based chemotherapeutic agents, such as paclitaxel, carboplatin and oxaliplatin. Examples of co-therapy or co-treatment include 5 mg/kg bevacizumab (Avastin^{®}) every two week with bolus-IFL or 10 mg/kg bevacizumab (Avastin^{®}) every 2 weeks with FOLFOX4 for metastatic colorectal cancer, 15 mg/kg bevacizumab (Avastin^{®}) every 3 weeks with caboplatis/paclitaxel for non-squamous non-small cell lung cancer, and 10 mg/kg bevacizumab (Avastin^{®}) every 2 weeks with paclitaxel for metastatic breast cancer. Further, the angiogenesis inhibitor to be administered may be administered as a co-therapy or a co-treatment with radiotherapy.

### 5. Kit

The present invention also relates to a diagnostic composition or kit comprising any of the mentioned oligonucleotides and optionally suitable means for detection.

The kit of the invention may advantageously be used for carrying out a method of the invention and could be, inter alia, employed in a variety of applications, e.g., in the diagnostic field or as a research tool. The parts of the kit of the invention can be packages individually in vials or in combination in containers or multicontainer units. Manufacture of the kit follows preferably standard procedures which are known to the person skilled in the art. The kit or diagnostic compositions may be used for detection of the one or more variant alleles in accordance with the herein-described methods of the invention, employing, for example, amplification techniques as described herein.

Accordingly, in a further embodiment of the present invention provides a kit useful for carrying out the methods herein described, comprising oligonucleotides or polynucleotides capable of determining the genotype of one or more SNPs. The oligonucleotides or polynucleotides may comprise primers and/or probes.

The present invention is further described by reference to the following non-limited figures and examples.

### Examples

### Example 1:

Genetic determination can influence sensitivity of the endothelium to VEGF. In accordance with this and in context of this invention, we explored genetic variability in underlying signaling pathways in order to discover predictive patterns for anti angiogenic treatment and the development of hypertension under this therapy regimen. In this analysis, the correlation of genetic variability in the VEGF-A signaling pathway with clinical outcome of patients with different advanced primary cancers was assessed in 5 different trials. All five were randomised parallel trials to investigate the efficacy and safety of BEV (bevacizumab) in subjects with metastatic colorectal cancer (NO16966), metastatic pancreatic cancer (AVITA), advanced or recurrent non-squamous non-small-cell lung cancer (AVAIL), metastatic renal cancer (AVOREN) and HER2-negative metastatic breast cancer (AVADO).

In all five trials, optional DNA biomarker sampling was included for SNP analysis. In total, germline DNA was available from 1346 patients. Common single nucleotide polymorphisms (SNPs) located in the hypoxia-inducible factor-1α and -2α, VEGF-A, its receptors (VEGFR-1 and -2) and other relevant genes were selected based on literature and using a SNP tagging approach (f≥0.1 and r² ≤ 0.8). 157 SNPs were successfully genotyped using MALDI-TOF mass spectrometry. Risk and survival estimates were calculated using Cox regression analyses. Two types of analyses were performed.
1. Correlation of genetic markers to PFS and OS.
2. Correlaton of genetic markers to hypertension, not classified as "unrelated to study drug"

The rs699946 (SEQ ID NO. 1) SNP, located in the VEGF-A promoter, was associated with improved PFS in bev-treated subjects with an allelic HR of 1.26 (95% CI 1.07-1.48, p=0.005). No effect was seen in placebo subjects, suggesting that rs699946 (SEQ ID NO. 1) may be a predictive marker for favourable outcome with bevacizumab treatment. Further, the rs11133360 (SEQ ID NO. 5) SNP, located in the VEGFR2, was associated with improved PFS in bevacizumab-treated subjects with an allelic HR of 1.15 (95% CI 1.02-1.30, p=0.02). In terms of OS, the rs12505758 (SEQ ID NO. 2) SNP in VEGFR2 was most significantly associated with improved OS in bev-treated pts (allelic HR 1.50, 95% CI 1.21-1.86, p=0.0002). No effects for rs12505758 (SEQ ID NO. 2) were seen in placebo pts.

Ten SNPs were associated with bevacizumab-induced hypertension (p<0.05), but none of these surpassed the threshold for multiple testing (p<0.0003). The two SNPs showing the strongest association (p<0.01) were: rs2305949 (SEQ ID NO. 3) in KDR (allelic OR 0.93, 95% CI 0.88-0.98, p=0.0067), and rs4444903 (SEQ ID NO. 4) in EGF (allelic OR 1.06, 95% CI 1.02-1.11, p=0.0052). Interestingly, rs2305949 (SEQ ID NO. 3) and rs4444903 (SEQ ID NO. 4) were closely linked to amino acid changes occurring on position 273 and 708 of KDR and EGF, suggesting that these changes may functionally affect both genes and thereby contribute to hypertension. Notably, rs11064560 in WNK1 was also associated with bevacizumab-induced hypertension (allelic OR 1.06, 95% CI 1.01-1.10, p=0.02), thereby supporting previous observations in a limited number of patients [Frey et al. J Clin Oncol 26: 2008 (May 20 suppl; abstr 11003)].

### PATIENTS AND METHODS

### Samples

All 5 trial protocols were approved by the institutional review board at each site and were conducted in accordance with the Declaration of Helsinki, current US Food and Drug Administration Good Clinical Practices, and local ethical and legal requirements. In total, 1346 subjects were genotyped. Among these subjects, 1225 were white and 121 were non-white. As non-white patients are genetically distinct from white patients and SNP frequencies may differ between both ethnic groups, non-white patients were omitted from further analysis. All patients provided separate written informed consent for genetic biomarker testing.

### Assessments

Patients were assessed according to the study protocol as described in the following references:
- AVITA: Van Cutsem et al., J. Clinc. Oncol. 27, 2231-7 (2009)
- AVAIL: Reck M, von Pawel J, Zatloukal P, et al. Phase III trial of cisplatin plus gemcitabine with either placebo or bevacizumab as first-line therapy for nonsquamous non-small-cell lung cancer: AVAiL. J Clin Oncol 2009;27(8):1227-34
- AVOREN: Escudier B, Pluzanska A, Koralewski P, Ravaud A, Bracarda S, Szczylik C, et al. Bevacizumab plus interferon alfa-2a for treatment of metastatic renal cell carcinoma: a randomised, double-blind phase III trial. Lancet. 2007;370(9605):2103-2111
- AVADO: Miles DW, et al., J Clin Oncol 2010a;28(20):3239-47
- NO16966: Saltz LB, Clarke S, Diaz-Rubio E, et al. Bevacizumab in combination with oxaliplatin-based chemotherapy as first-line therapy in metastatic colorectal cancer: a randomized phase III study. J Clin Oncol 2008;26(12):2013-2019

### Single Nucleotide Polymorphism Selection

Two marker panels were considered for analysis: The Roche panel and Leuven panel. The Roche panel consists of 35 genetic polymorphisms selected by literature review for potential relevance to BEV treatment. The panel is made up of SNPs and repeat polymorphisms lying in the following genes: VEGFA, NOS3, FLT1 (VEGFR1), KDR (VEGFR2), WNK1, IL8, IL8R and IFNAR2. The Leuven panel consists of 186 tag SNPs from the VEGF signalling cascade or from candidate genes for known side-effects, hypertension or thrombosis and includes the following genes: the VEGF ligand, the VEGF homologues (placental growth factor or PlGF, VEGF-B and -C, as well as VEGF-D or FlGF), the VEGF receptor-2 (KDR or VEGFR-2) and VEGF receptor-1 (FLT1 or VEGFR-1). Genomic sequences 5 kb upstream of the translation start site up to 3' poly-A adenylation site of each gene, were used to select SNPs from the HapMap database (HapMap Data Rel 24/phaseII Nov08, on NCBI B36 assembly, dbSNP b126). Tagging SNPs were selected using the Tagger (Pe'er, I., et al., Nat. Genet. 38, 663-7 (2006)) as provided in the HAPLOVIEW software package (Barrett, J.C., et al., Bioinformatics. 21, 263-5 (2005)). Only SNPs occurring commonly, i.e., with a minor allele frequency f≥0.1 and a minimum r² threshold ≥0.8 were considered. In total, 167 tagging SNPs were selected following these criteria. Additionally, 11 SNPs located in exonic sequences and inducing non-synonymous amino-acid changes at a frequency f≥0.1 were selected from the dbSNP database, as well as 4 SNPs in VEGF (rs699947, rs833061, rs2010963 and rs3025039), 1 SNP in VEGFR-1 (rsTP53_R-1) and 1 SNP in VEGFR-2 (rs2071559), which previously have been reported to affect function or expression of these genes.

There is some overlap between markers in the two panels, and the size of the panels has increased slightly over the duration of the six trials, so fewer markers are available for earlier trials. The current meta-analysis is confined to markers for which genotyping was conducted in at least two trials under study.

Four marker sets were defined as follows:
- 'All Markers' consists of all markers assayed for which at least one genotype was obtained.
- 'Roche Markers' consists of markers from the Roche panel, which passed quality checks (see below) and had frequency greater than 1% in white subjects.
- 'Leuven Efficacy Markers' consists of those markers from the Leuven panel, which lie in loci implicated in the VEGF pathway, which passed quality checks (see below) and which had frequency greater than 1% in white subjects.
- 'Leuven Safety Markers' consists of those markers from the Leuven panel which lie in candidate genes for involvement in hypertension or thrombosis, which passed quality checks (see below) and which had frequency greater than 1% in white subjects.

### Genotyping

Peripheral blood was sampled in K2EDTA plastic Vacutainer tubes. After centrifugation, germline DNA was extracted from the precipitated leucocyte cell fraction according to standard procedures.

For SNPs of the Roche panel, Genotyping was carried out in a blinded manner at the Roche Translational Research Sciences Genetics laboratories (Basel, Switzerland) using Allele Specific PCR amplification, Sanger sequencing and Fragment Analysis platforms (AVAIL, AVITA, AVOREN, AVADO, NO16966).

For SNPs of the Leuven panel, genotyping was carried out in a blinded manner at the Vesalius Research Center (Leuven, Belgium) using the Sequenom iPLEX platform (Sequenom Inc, San Diego, CA, USA). Any SNP that failed to provide a robust genotype in the first genotyping round was redesigned using a different set of polymerase chain reaction primers and retested. Overall, 157 (85.3%) were successfully genotyped with an overall success rate of 98.5%. The 27 SNPs that also failed the second design were considered failures.

The following amplification primers were designed for SNPs rs699946 (SEQ ID NO. 1), rs12505758 (SEQ ID NO. 2), rs2305949 (SEQ ID NO. 3), rs4444903 (SEQ ID NO. 4) and rs11133360 (SEQ ID NO. 5). For rs699946 (SEQ ID NO. 1), ACGTTGGATGCTACCACTAGTGTTGGCTTG (SEQ ID NO. 6) and ACGTTGGATGTGAGCTCCACACTGCCTTC (SEQ ID NO. 7) were used. For SNP rs12505758 (SEQ ID NO. 2), ACGTTGGATGCTTTACTCTGCCAAATCTATG (SEQ ID NO. 8) and ACGTTGGATGGCTAATAAGCTTATACATTTG (SEQ ID NO. 9) were used. For rs2305949 (SEQ ID NO. 3), ACGTTGGATGATCCTATACCCTAGAGCAAG (SEQ ID NO. 10) and ACGTTGGATGATCTGTGCAAAGTTATAGGC (SEQ ID NO. 11) were used. For rs4444903 (SEQ ID NO. 4), ACGTTGGATGTCTTCTTTCAGCCCCAATCC (SEQ ID NO. 12) and ACGTTGGATGAAGAAAGGAAGAACTGATGG (SEQ ID NO. 13) were used. For rs11133360 (SEQ ID NO. 5), ACGTTGGATGTTTCACATTGCTATGCCCAA (SEQ ID NO. 14) and ACGTTGGATGCTCTTTCTTCACTTTGACTG (SEQ ID NO. 15) were used.

The following unextended primers (probes) were designed for SNPs rs699946 (SEQ ID NO. 1), rs12505758 (SEQ ID NO. 2), rs2305949 (SEQ ID NO. 3), rs4444903 (SEQ ID NO. 4) and rs11133360 (SEQ ID NO. 5). For rs699946 (SEQ ID NO. 1), ATTAGTCAATTCTCTGACAGAGACA (SEQ ID NO. 16) was used. For rs12505758 (SEQ ID NO. 2), TTACTCTGCCAAATCTATGATGCCA (SEQ ID NO. 17) was used. For rs2305949 (SEQ ID NO. 3), CTAGAGCAAGTAAATTGAAAAAA (SEQ ID NO. 18) was used. For rs4444903 (SEQ ID NO. 4), GCATCTCCAATCCAAGGGTTGT (SEQ ID NO. 19) was used. For rs11133360 (SEQ ID NO. 5), CACATTGCTATGCCCAACACATC (SEQ ID NO. 20) was used.

### Quality checking

Data were checked for quality as follows:
- Uniformity of assay strand was ensured
- Levels of missing data were summarised
- Markers with minor allele frequency (MAF<1%) were excluded
- Markers failing a test of homogeneity of allele frequency were excluded
- Tests of Hardy Weinberg Equilibrium (HWE) were conducted to assist interpretation

After quality check, 25 Roche Markers, 133 Leuven Efficacy Markers and 22 Leuven Safety Markers were subjected to pooled association analysis.

### Statistical analysis

A pooled analysis of individual patient data, stratified by study was applied to all markers with homogeneous frequency. Candidate markers for efficacy were tested for association to PFS and OS using Cox Proportional Hazards Regression. Candidate markers for safety were tested for association to Hypertension using logistic regression. The primary analysis involved white BEV-treated subjects from the ITT (for efficacy endpoints) and SP (hypertension), as appropriate. Adjustments were made in all association tests for the following covariates: Region, study and dose and background chemotherapy regimen. A subset of the following variables, selected by endpoint using backwards stepwise regression, were adjusted for as well: ECOG performance status (0 vs. 1), gender (male vs. female), age, LDH, alkaline phosphatase level (within normal range vs. above normal range), serum albumin (<2.9g/dL vs. >=2.9g/dL) and baseline number of metastatic sites (>2 vs. <=2). In order to investigate whether any detected associations reflected temporal rather than treatment effects, association testing was also conducted in white placebo-treated subjects. In order to further characterise any detected associations, Genotype x Treatment interaction analysis was conducted in white subjects.

### Data

26 Roche Markers, 136 Leuven Efficacy Markers and 22 Leuven Safety Markers passed quality checking and were subjected to homogeneity analysis. Table 1 shows the number of subjects to be incorporated into the meta-analysis. It is noted that 3 subjects in the ITT were not in the SP and 3 subjects had missing values for randomised weekly dose (RNDWD).

**Table 1: Subject sets and sizes for analysis**

| **Analysis Population** | **Count** |
|---|---|
| *PGx-ITT-All Arms-All Ethnicities* | 1346 |
| *PGx-ITT-All Arms-White* | 1225 |
| *PGx-ITT-BEV-All Ethnicities* | 668 |
| *PGx-ITT-BEV-White* | 629 |
| *PGx-ITT-PBO-White* | 593 |
| *PGx-SP-All Arms-White* | 1223 |
| *PGx- SP -BEV-All Ethnicities* | 667 |
| *PGx- SP -BEV- White* | 628 |
| *PGx- SP -PBO-White* | 592 |

### Clinical Characteristics of genetic patient population

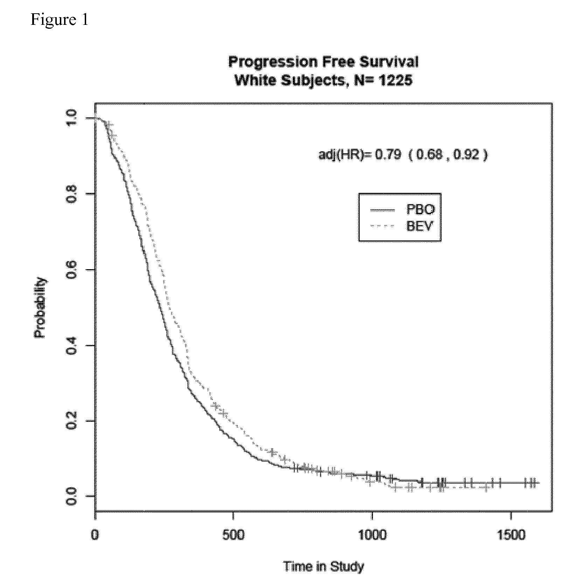

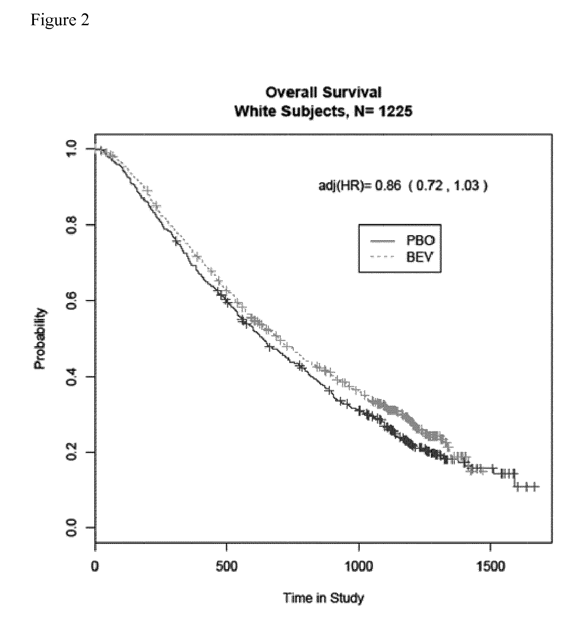

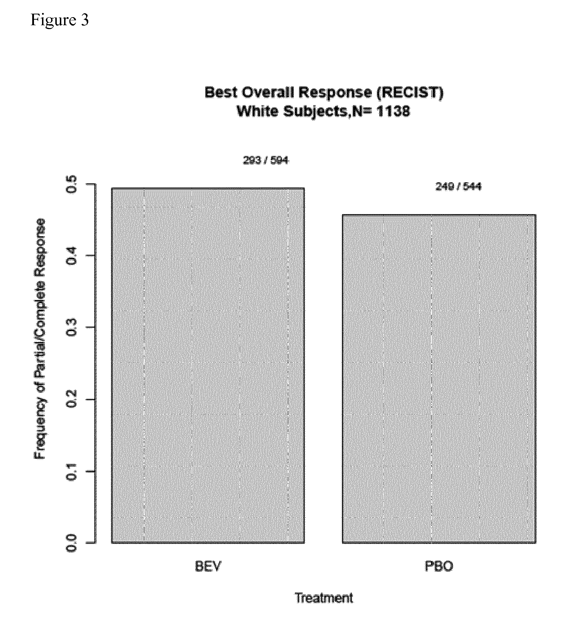

Demographic and endpoint characteristics are tabulated in Table 2 by clinical trial and overall for subjects in *PGx-ITT-BEV-All Ethnicities.* Endpoint distributions are portrayed graphically in Figure 1-4.

**Table 2: Summary of demographic and endpoint data**

| | **AVAIL** | **AVOREN** | **AVITA** | **AVADO** | | **All** |
|---|---|---|---|---|---|---|
| | **BO17704** | **BO17705** | **BO17706** | **BO17708** | **NO16966** | |
| **Cancer-type** | NSCLC | Renal | Pancreatic | Breast | Colorectal | |
| N | 119 | 108 | 161 | 350 | 610 | 1348 |
| BEV | 83 | 59 | 79 | 238 | 210 | 669 |
| PBO | 36 | 49 | 81 | 110 | 400 | 676 |
| Male | 0.71 | 0.72 | 0.64 | 0 | 0.61 | 0.47 |
| Age (yr) (SD) | 57.39 (9.85) | 59.7 (10.57) | 61.7 (9.59) | 54.73 (10.84) | 59.56 (11.46) | 58.38 (11.13) |
| White | 0.92 | 0.99 | 0.95 | 0.96 | 0.85 | 0.91 |
| OS (Median) | 424 | 1011 | 213 | 873.5 | 636 | 614 |
| Cens. (OS) | 0.36 | 0.47 | 0.09 | 0.37 | 0.19 | 0.26 |
| PFS (Median) | 197 | 413 | 150 | 256 | 267.5 | 250 |
| Cens. (PFS) | 0.03 | 0.11 | 0.04 | 0.07 | 0.04 | 0.05 |
| BOR (PR/CR) | 0.42 | 0.37 | 0.14 | 0.49 | 0.51 | 0.44 |
| Hypertension | 0.21 | 0.2 | 0.14 | 0.15 | 0.08 | 0.13 |

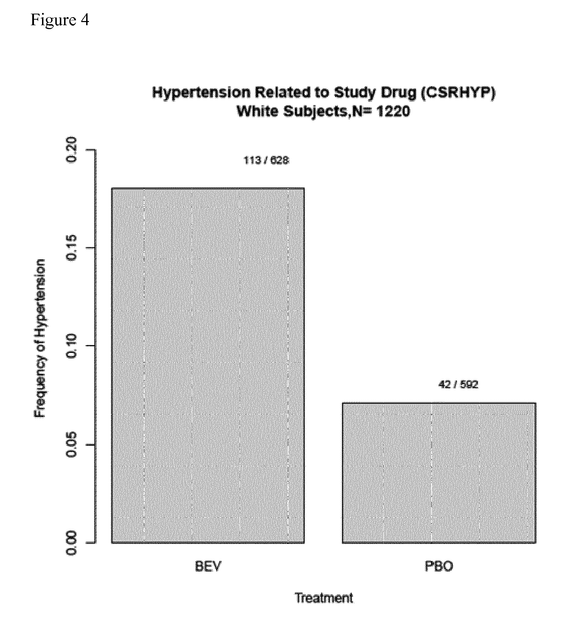

Clinical data was available for 1348 subjects from 5 trials, with approximately equal numbers overall on treated and placebo. In contrast to the other trials, no males participated in BO17708 (AVADO), which was a trial of advanced breast cancer. The age distributions and the proportions white were broadly homogeneous, except for a slightly lower proportion of white subjects in the largest trial, NO16966. The median lengths of OS and PFS varied widely as did the rates of censoring. As expected, censoring for OS was far greater than for PFS and in statistical terms, the effect will be to reduce power to detect association to OS compared to PFS. Rates of BOR were 49% in BEV-treated subjects and 46% in PBO-treated subjects. Rates of hypertension were 18% in BEV-treated subjects and 7% in PBO-treated subjects (Figure 4).

For PFS there were 592 events and for OS, there were 438 events out of 629 white BEV-treated subjects in the PGx-ITT. For Hypertension, there were 113 events out of a total of 628 white BEV-treated subjects in PGx-SP. Table 3 shows the number of white subjects to be incorporated into the meta-analysis.

**Table 3: Subject counts for pharmacogenetic meta-analysis of white subjects**

| **Population** | **Treatment** | **Count** |
|---|---|---|
| ITT | BEV | 629 |
| ITT | PBO | 593 |
| SP | BEV | 628 |
| SP | PBO | 592 |

| | | |
|---|---|---|
| ITT: intent-to-treat population SP: safety population | | |

### Results Efficay

### Progression Free Survival

### Analysis in the sub-group of patients treated with Bevacizumab

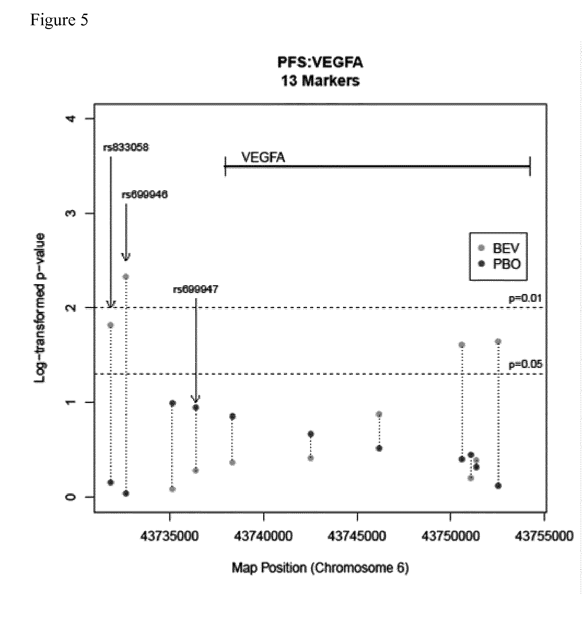

The strongest association in VEGF-A with PFS was for rs699946 (SEQ ID NO. 1) (p=0.005). Although the result would not be significant after adjustment for multiple-testing, it provides consistent upstream signal with marker rs699947, published by Schneider et al. J Clin Oncol 2008 26:4672. A scatter plot of association across the whole gene is given in Figure 5.

| **Marker** | **Chr** | **BP** | **MAF** | **N** | **HR** | **95% CI** | **p-value** | **Gene** |
|---|---|---|---|---|---|---|---|---|
| rs699946 | Chr6 | 43732669 | 0.18 | 542 | 1.26 | (1.07,1.48) | 0.0047 | VEGFA |

Relative to AA carriers, HR for rs699946 (SEQ ID NO. 1) AG carriers was 1.26 (95% CI 1.07-1.48, p=0.005). This means that each additional G allele was associated with 27% increase in risk of progression or death. No effect was seen in placebo subjects, suggesting that rs699946 (SEQ ID NO. 1) may be a predictive marker for favourable outcome with bevacizumab treatment.

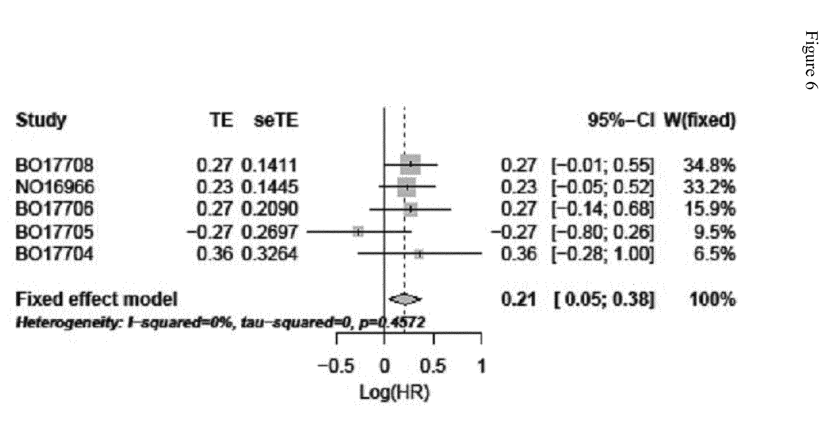

As shown in Figure 6, consistent effects for rs699946 (SEQ ID NO. 1) were seen for all studies except AVOREN/BO17705 (renal cancer), the smallest study under consideration.

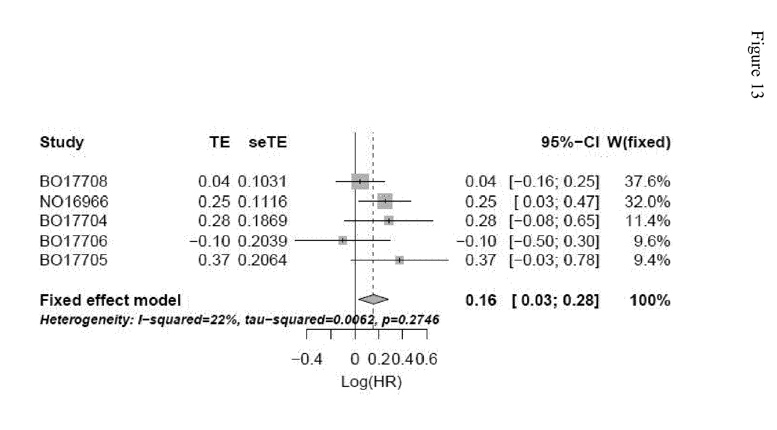

Further, the rs11133360 (SEQ ID NO. 5) was weakly associated (p=0.02) to PFS (Figure 13).

| **Marker** | **Chr** | **BP** | **MAF** | **N** | **HR** | **95% CI** | **p-value** | **Gene** |
|---|---|---|---|---|---|---|---|---|
| rs11133360 | Chr4 | 55982752 | 0.45 | 579 | 1.15 | (1.02,1.30) | 0.02 | KDR |

Relative to TT carriers, HR for rs11133360 (SEQ ID NO. 5) CT carriers was 1.15 (95% CI 1.02-1.30, p=0.02). This means that each additional C allele was associated with 15% increase in risk of progression or death.

### Overall Survival Analysis

### Analysis in the sub-group of patients treated with Bevacizumab

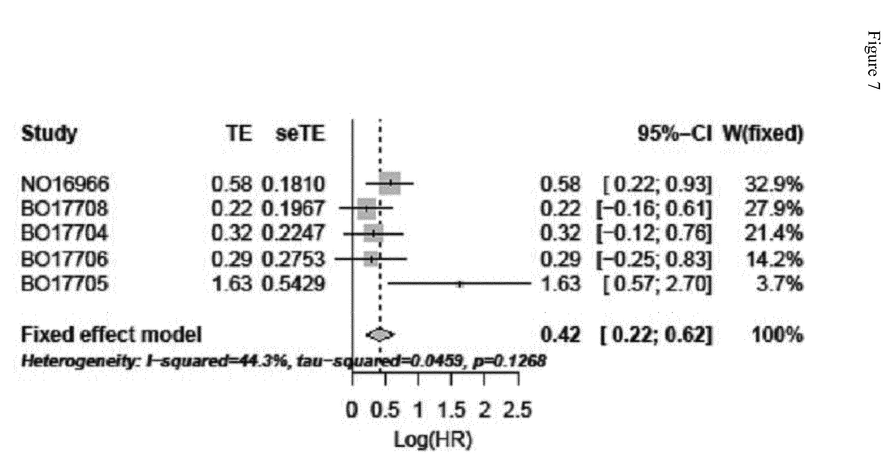

In association analysis for OS, 6/133 markers had p<0.05 in white treated subjects. One of these, rs12505758 (SEQ ID NO. 2) is significant after Bonferroni adjustment for 158 tests. The marker is an intronic SNP in KDR (Kinase Insert Domain Receptor; VEGFR2; FLK1) and it is in LD (r2=0.31) with another intronic SNP, rs1531289 in the gene (source: HapMap release 22; the marker not available in HapMap v3 release 2). The marker is not associated in white placebo-treated subjects therefore it may be said to have predictive as opposed to prognostic qualities. An inspection of the forest plot (Figure 7) shows that the effect is driven primarily by NO16966 (colorectal cancer) and BO17705 (AVOREN; renal cancer). The effect is either weak or absent in the other three studies.

| **Marker** | **Chr** | **BP** | **MAF** | **N** | **HR** | **95% CI** | **p-value** | **Gene** |
|---|---|---|---|---|---|---|---|---|
| rs12505758 | Chr4 | 55966898 | 0.12 | 581 | 1.50 | (1.21, 1.86) | 2.00E-04 | KDR |

Relative to TT carriers, HR for rs12505758 (SEQ ID NO. 2) TC carrier was (allelic HR 1.50, 95% CI 1.21-1.86 (, p=0.0002). This means that each additional C allele was associated with 50% increase in risk of death. No effects for rs12505758 (SEQ ID NO. 2) were seen in placebo patients.

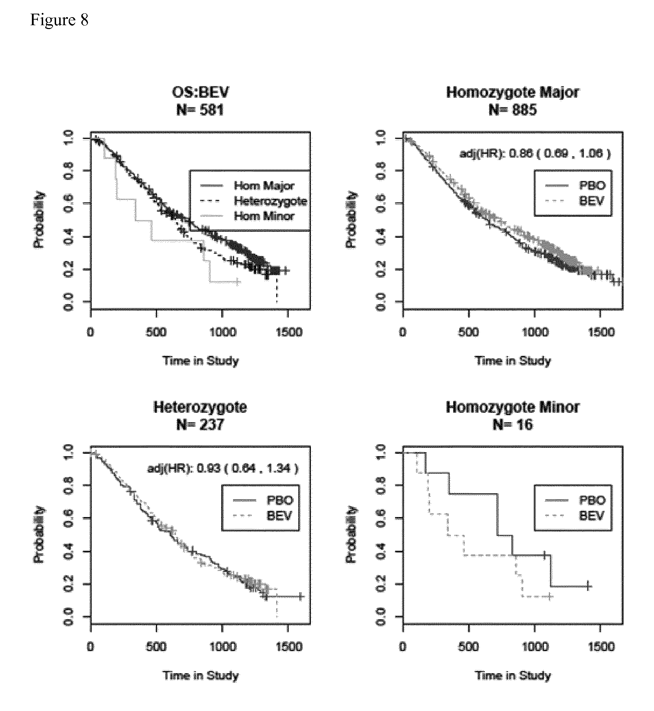

The Kaplan Meier plots show increasing estimated hazard ratio with increasing number of copies of the minor allele (Figure 8).

### Additional information on SNPs

Since the rs699946 (SEQ ID NO. 1) SNP is located in the VEGF promoter, we examined its effect on VEGF-A expression in human plasma samples. We found that GG carriers have a 27% increased median VEGF expression compared to AG and AA (wildtype) carriers. The minor allele of rs699946 (SEQ ID NO. 1) was in linkage with the minor allele of rs699947 (D' 0.98; r²=0.23), which is another VEGF promoter SNP previously shown to associate with response to bevacizumab therapy [Schneider et al, J Clin Oncol 2008 26:4672]. Furthermore, rs699946 (SEQ ID NO. 1) is also in linkage with rs833058 (-6589 C>T), which emerged as the second hit in VEGF for PFS in the meta-analysis (D'=0.95, r²=0.35).

Conclusion of additional research: We demonstrated that the G allele of rs699946 (SEQ ID NO. 1) in the VEGF-A promoter is associated with an increased plasma VEGF-A expression and that rs699946 (SEQ ID NO. 1) is in linkage with rs699947, another VEGF-A promoter SNP previously shown to associate with response to bevacizumab by Schneider et al.

The rs11133360 (SEQ ID NO. 5) in VEGFR-2 is an intronic SNP located between the exons that encode the extracellular domains of the VEGFR-2 protein. We found that HUVECs homozygous for the minor C allele have increased proliferation upon VEGF stimulation compared to CT and TT carriers. Importantly, rs11133360 is also in strong linkage with rs2305948 (D'= 1), a nonsynonymous SNP that induces the Val297Ile substitution and which has been reported to affect the binding of VEGF to VEGFR-2.

### Results Hypertension

The two SNPs showing the strongest association (p<0.01) were: rs2305949 (SEQ ID NO. 3) in KDR (allelic OR 0.93, 95% CI 0.88-0.98, p=0.0067), rs4444903 (SEQ ID NO. 4) in EGF (allelic OR 1.06, 95% CI 1.02-1.11, p=0.0052); see table 4, but none of these surpassed the threshold for multiple testing (p<0.0003). Interestingly, rs2305949 (SEQ ID NO. 3) and rs4444903 (SEQ ID NO. 4) were closely linked to amino acid changes occurring on position 273 and 708 of KDR and EGF, suggesting that these changes may functionally affect both genes and thereby contribute to hypertension.

**Table 4 Association results for SNP markers with Hypertension**

| **Marker** | **Chr** | **BP** | **MAF** | **N** | **OR** | **95% CI** | **p-value** | **Gene** |
|---|---|---|---|---|---|---|---|---|
| rs2305949 | 4 | 55980456 | 0.22 | 578 | 0.93 | (0.88,0.98) | 0.0067 | KDR |
| rs4444903 | 4 | 111053559 | 0.43 | 609 | 1.06 | (1.02,1.11) | 0.005 | EGF |

| | | | | | | | | |
|---|---|---|---|---|---|---|---|---|
| Chr = chromosome; MAF: Minor Allele Freqeuncy; OR = Odds ratio | | | | | | | | |

### rs2305949 (SEQ ID NO. 3) (KDR)

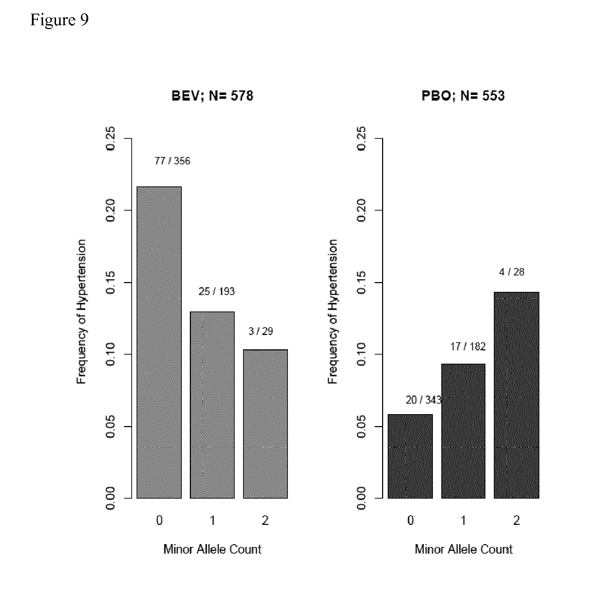

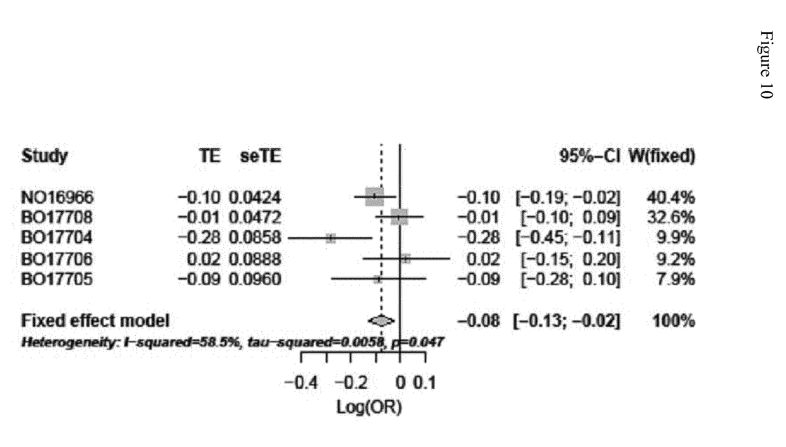

As shown in Figure 9, higher frequency of hypertension was seen for the CC carrier. Figure 10 shows that three studies, (NO16966, AVAIL/BO 17704 and AVOREN/BO17705) drive the association. The forest plot for white placebo-treated subjects (not shown) shows the mean effect across studies is weakly in the opposite direction, so the marker may be concluded to have predictive characteristics.

### rs4444903 (SEQ ID NO. 4) (EGF)

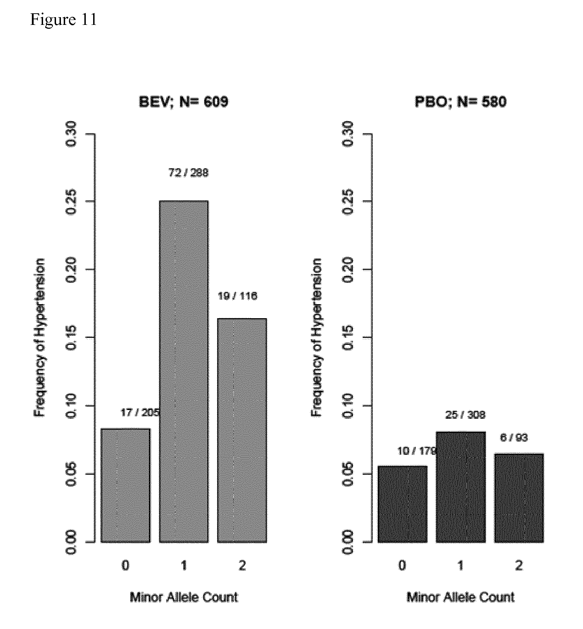

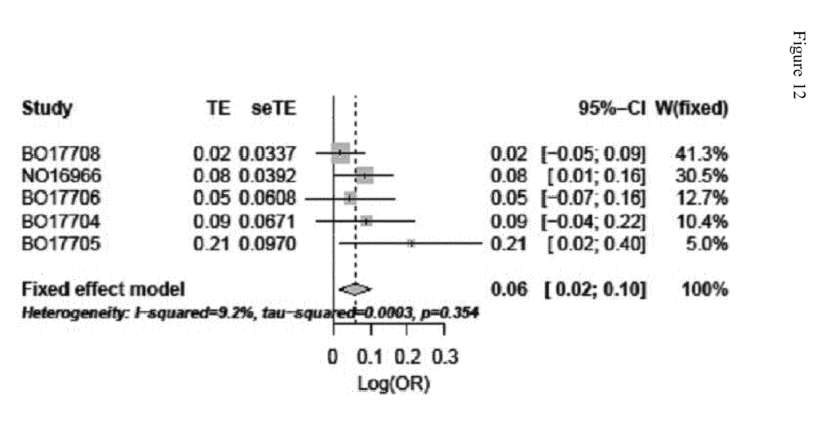

As shown in Figure 11, higher frequency of hypertension was seen for the GA carrier, with lowest frequency for the AA carriers, while the frequency of the GG carrier was in between. For marker rs4444903 (SEQ ID NO. 4) in EGF, examination of the forest plot (Figure 12) shows reasonable consistency across studies, with weakest effect observed inBO17708/ AVADO (breast cancer). The forest plot for subjects in the placebo arm (not shown) shows that the marker has predictive as opposed to prognostic characteristics.

## Claims

1. A method of determining susceptibility of a patient to developing hypertension associated with a therapy by an angiogenesis inhibitor comprising bevacizumab or an antibody that binds essentially the same epitope on VEGF as bevacizumab, said method comprising:
(a) determining in a sample derived from a patient suffering from cancer the genotype at polymorphism rs2305949 (SEQ ID NO. 3), and
(b) identifying a patient as more or less susceptible to developing hypertension associated with a therapy by an angiogenesis inhibitor comprising bevacizumab or an antibody that binds essentially the same epitope on VEGF as bevacizumab based on said genotype, wherein the presence of CC genotype at polymorphism rs2305949 (SEQ ID NO. 3) indicates that said patient is more susceptible to developing hypertension than a patient having a genotype of CT or TT at polymorphism rs2305949 (SEQ ID NO. 3), or the presence of CT or TT genotype at polymorphism rs2305949 (SEQ ID NO. 3) indicates that said patient is less susceptible to developing hypertension than a patient having a genotype of CC at polymorphism rs2305949 (SEQ ID NO. 3).

2. The method of claim 1, wherein the therapy further comprises a chemotherapeutic agent or chemotherapy regimen.

3. The method of claim 2, wherein the chemotherapeutic agent or chemotherapy regimen comprises one or more agents selected from the group consisting of taxanes, interferon alpha, 5-fluorouracil, leucovorin, irinotecan, gemcitabine-erlotinib and platinum-based chemotherapeutic agents.

4. The method of any one of claims 1 to 3, wherein the cancer is pancreatic cancer, renal cell cancer, colorectal cancer, breast cancer or lung cancer.

5. The method of any one of claims 1 to 4, wherein the sample is a blood sample.

6. The method of any one of claims 1 to 5, wherein the genotype is determined by means of MALDI-TOF mass spectrometry.

7. A pharmaceutical composition comprising an angiogenesis inhibitor as defined in claims 1 to 6 for use in the treatment of a patient in need thereof, wherein said patient is determined in accordance with the method of any one of claims 1 to 6 to be less susceptible to developing hypertension associated with a therapy comprising the angiogenesis inhibitor.

8. An angiogenesis inhibitor comprising bevacizumab or an antibody that binds essentially the same epitope on VEGF as bevacizumab for use in reducing the risk of developing hypertension associated with a therapy comprising an angiogenesis inhibitor comprising bevacizumab or an antibody that binds essentially the same epitope on VEGF as bevacizumab, said method comprising:
(a) determining in a sample derived from a patient suffering from cancer the genotype at polymorphism rs2305949 (SEQ ID NO. 3); and
(b) identifying a patient as less susceptible to developing hypertension associated with a therapy by an angiogenesis inhibitor comprising bevacizumab or an antibody that binds essentially the same epitope on VEGF as bevacizumab, wherein the presence of CT or TT genotype at polymorphism rs2305949 (SEQ ID NO. 3) indicates that said patient is less susceptible to developing hypertension than a patient having a genotype of CC at polymorphism rs2305949 (SEQ ID NO. 3), whereby said angiogenesis inhibitor is administered to a patient with the CT or TT genotype at polymorphism rs2305949 (SEQ ID NO. 3) identified as less susceptible to developing hypertension in accordance with (b).

9. The angiogenesis inhibitor for the use of claim 8, wherein the therapy further comprises a chemotherapeutic agent or chemotherapy regimen.

10. The angiogenesis inhibitor for the use of claim 9, wherein the chemotherapeutic agent or chemotherapy regimen comprises one or more agents selected from the group consisting of taxanes, interferon alpha, 5-fluorouracil, leucovorin, irinotecan, gemcitabine-erlotinib and platinum-based chemotherapeutic agents.

11. The angiogenesis inhibitor for the use of any one of claims 8 to 10 wherein the cancer is pancreatic cancer, renal cell cancer, colorectal cancer, breast cancer or lung cancer.

12. The angiogenesis inhibitor for the use of any one of claims 8 to 11, wherein the sample is a blood sample.

13. The angiogenesis inhibitor for the use of any one of claims 8 to 12, wherein the genotype is determined by means of MALDI-TOF mass spectrometry.


<110>  F. Hoffmann - La Roche AG
        Life Science Research Partners vzw
        VIB vzw

<120>  Method for predicting risk of hypertension associated with
        anti-angiogenesis therapy

<130>  30616

<150>  EP11179500.1
<151>  2011-08-31

<160>  20

<170>  PatentIn version 3.5

<210>  1
<211>  101
<212>  DNA
<213>  Homo sapiens

<400>  1
agctggacct accactagtg ttggcttgtt taaattctct gacagagaca ractgtcccg      60

gggggcgggg aaggcagtgt ggagctcacc ccctgaggga g                          101


<210>  2
<211>  101
<212>  DNA
<213>  Homo sapiens

<400>  2
ctaataagct tatacatttg aaaatatgtc aacatatctc gatgcaatac ytggcatcat      60

agatttggca gagtaaaggg accaaaatct agactttgag a                          101


<210>  3
<211>  101
<212>  DNA
<213>  Homo sapiens

<400>  3
tcagaaccac atcataaatc ctatacccta gagcaagtaa attgaaaaaa yagaacatga      60

gagagcaaat aagcctataa ctttgcacag atttattttt t                          101


<210>  4
<211>  101
<212>  DNA
<213>  Homo sapiens

<400>  4
gaggaatcgt atctccatat ttcttctttc agccccaatc caagggttgt rgctggaact      60

ttccatcagt tcttcctttc tttttcctct ctaagccttt g                          101


<210>  5
<211>  101
<212>  DNA

<213>  Homo sapiens

<400>  5
gcttgaaatc tcagagagat aacatttcac attgctatgc ccaacacatc ycatcaagca      60

ttctcagagt gcttcataat agtcagtcaa agtgaagaaa g                         101


<210>  6
<211>  30
<212>  DNA
<213>  Artificial Sequence

<220>
<223>  an artificially synthesized primer for PCR

<400>  6
acgttggatg ctaccactag tgttggcttg                                      30


<210>  7
<211>  29
<212>  DNA
<213>  Artificial Sequence

<220>
<223>  an artificially synthesized primer for PCR

<400>  7
acgttggatg tgagctccac actgccttc                                       29


<210>  8
<211>  31
<212>  DNA
<213>  Artificial Sequence

<220>
<223>  an artificially synthesized primer for PCR

<400>  8
acgttggatg ctttactctg ccaaatctat g                                    31


<210>  9
<211>  31
<212>  DNA
<213>  Artificial Sequence

<220>
<223>  an artificially synthesized primer for PCR

<400>  9
acgttggatg gctaataagc ttatacattt g                                    31


<210>  10
<211>  30
<212>  DNA
<213>  Artificial Sequence

<220>
<223>  an artificially synthesized primer for PCR

<400>  10

acgttggatg atcctatacc ctagagcaag                                        30

<210>  11
<211>  30
<212>  DNA
<213>  Artificial Sequence

<220>
<223>  an artificially synthesized primer for PCR

<400>  11
acgttggatg atctgtgcaa agttataggc                                        30

<210>  12
<211>  30
<212>  DNA
<213>  Artificial Sequence

<220>
<223>  an artificially synthesized primer for PCR

<400>  12
acgttggatg tcttctttca gccccaatcc                                        30

<210>  13
<211>  30
<212>  DNA
<213>  Artificial Sequence

<220>
<223>  an artificially synthesized primer for PCR

<400>  13
acgttggatg aagaaaggaa gaactgatgg                                        30

<210>  14
<211>  30
<212>  DNA
<213>  Artificial Sequence

<220>
<223>  an artificially synthesized primer for PCR

<400>  14
acgttggatg tttcacattg ctatgcccaa                                        30

<210>  15
<211>  30
<212>  DNA
<213>  Artificial Sequence

<220>
<223>  an artificially synthesized primer for PCR

<400>  15
acgttggatg ctctttcttc actttgactg                                        30

<210>  16
<211>  25

<212>  DNA
<213>  Artificial Sequence

<220>
<223>  an artificially synthesized probe

<400>  16
attagtcaat tctctgacag agaca                                            25


<210>  17
<211>  25
<212>  DNA
<213>  Artificial Sequence

<220>
<223>  an artificially synthesized probe

<400>  17
ttactctgcc aaatctatga tgcca                                            25


<210>  18
<211>  23
<212>  DNA
<213>  Artificial Sequence

<220>
<223>  an artificially synthesized probe

<400>  18
ctagagcaag taaattgaaa aaa                                              23


<210>  19
<211>  22
<212>  DNA
<213>  Artificial Sequence

<220>
<223>  an artificially synthesized probe

<400>  19
gcatctccaa tccaagggtt gt                                               22


<210>  20
<211>  23
<212>  DNA
<213>  Artificial Sequence

<220>
<223>  an artificially synthesized probe

<400>  20
cacattgcta tgcccaacac atc                                              23